# EUROPEAN PATENT APPLICATION

(11) **EP 0 636 626 A1**
(43) Date of publication of application: **01.02.1995**
(21) Application number: 94202083.5
(22) Date of filing: 18.07.1994
(51) Int. Cl.: C07D 487/04, A61K 31/505

(54) **Pyrazolopyrimidine Derivatives**

(30) Priority: 20.07.1993 GB 9315017
(71) Applicant: LABORATOIRES GLAXO SA, F-75016 Paris (FR)
(72) Inventor: Dumaitre, Bernard Andre, Laboratoires Glaxo SA, F-91940 Les Ulis (FR); Dodic, Nerina, Laboratoires Glaxo SA, F-91940 Les Ulis (FR)
(74) Representative: Caffin, Lee

(57) **Abstract**

Pyrazolo[3,4-d]pyrimidin-4-one derivatives of formula (I) 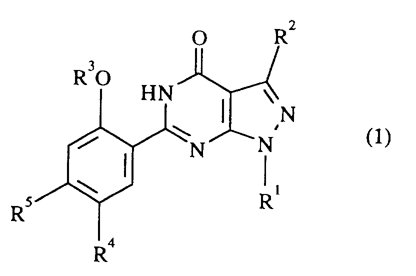
and salts and solvates thereof are described, in which:
R¹ represents arylmethyl or C₁₋₆alkyl optionally substituted by one or more fluorine atoms;
R² represents methyl;
R³ represents C₂₋₄alkyl;
R⁴ represents nitro, cyano, C₁₋₆alkoxy, C(=X)NR⁶R⁷, NR⁸R⁹, (CH₂)ₘNR¹⁰C(=Y)R¹¹ or a 5-membered heterocyclic ring selected from thienyl, thiazolyl and 1,2,4-triazolyl each ring optionally substituted by a C₁₋ ₄alkyl or aryl group; or when R¹ is arylmethyl or C₁₋₆alkyl substituted by one or more fluorine atoms then R⁴ may also represent hydrogen;
R⁵ represents hydrogen or C₁₋₆alkyl;
R⁶ represents hydrogen or C₁₋₆alkyl;
R⁷ represents hydrogen, amino, hydroxyl, C₁₋₆alkyl, aryl or arylC₁₋₄alkyl;
R⁸ represents hydrogen or C₁₋₆alkyl;
R⁹ represents hydrogen, C₁₋₆alkyl, SO₂R¹², CO₂R¹², C(=NCN)SR¹² or C(=NCN)NR¹³R¹⁴;
R¹⁰ represents hydrogen or C₁₋₆alkyl;
R¹¹ represents C₁₋₆alkyl optionally substituted by one or more halogen atoms, or R¹¹ represents aryl, arylC₁₋₄alkyl, thienyl, NR¹⁵R¹⁶, CH₂NR¹⁷R¹⁸ or R¹⁰ and R¹¹ together represent -A(CH₂)ₙ-;
R¹² represents C₁₋₆alkyl, aryl or arylC₁₋₄alkyl;
R¹³ represents hydrogen or C₁₋₆alkyl;
R¹⁴ represents hydrogen, C₁₋₆alkyl, aryl, arylC₁₋₄alkyl or R¹³ and R¹⁴ together with the nitrogen atom to which they are attached form a morpholine, piperazine or N-C₁₋₄alkylpiperazine ring;
R¹⁵ represents hydrogen or C₁₋₆alkyl or R¹⁰ and R¹⁵ together represent -A(CH₂)ₙ-;
R¹⁶ represents hydrogen, C₁₋₆alkyl, aryl, arylC₁₋₄alkyl, CO₂R¹², CH₂CO₂R¹² or R¹⁵ and R¹⁶ together with the nitrogen atom to which they are attached form a morpholine, piperazine or N-C₁₋₄alkylpiperazine ring;
R¹⁷ represents hydrogen or C₁₋₆alkyl;
R¹⁸ represents hydrogen, C₁₋₆alkyl, aryl, arylC₁₋₄alkyl, COR¹² or R¹⁷ and R¹⁸ together with the nitrogen atom to which they are attached form a morpholine, piperazine or N-C₁₋₄alkylpiperazine ring;
A represents CH₂ or C=O;
m represents zero or 1;
n represents 1,2 or 3;
X represents S or NH, or when R⁷ represents amino then X may also represent O;
Y represents O or S; for use in therapy.

These compounds are potent and selective inhibitors of cyclic guanosine 3',5'-monophosphate specific phosphodiesterase (cGMP specific PDE) and are useful in a variety of therapeutic areas, including the treatment of cardiovascular disorders.

## Description

This invention relates to a series of pyrazolo[3,4-d]pyrimidin-4-one derivatives, to processes for their preparation, pharmaceutical compositions containing them, and their use as therapeutic agents. In particular, the invention relates to pyrazolo[3,4-d]pyrimidin-4-one derivatives which are potent and selective inhibitors of cyclic guanosine 3',5'-monophosphate specific phosphodiesterase (cGMP specific PDE) having utility in a variety of therapeutic areas where such inhibition is thought to be beneficial, including the treatment of cardiovascular disorders.

Thus, according to a first aspect, the present invention provides compounds of formula (I) 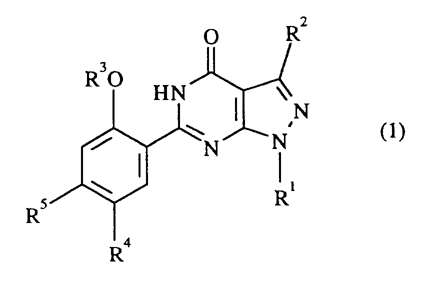
and salts and solvates (e.g. hydrates) thereof, in which:
R¹ represents arylmethyl or C₁₋₆alkyl optionally substituted by one or more fluorine atoms;
R² represents methyl;
R³ represents C₂₋₄alkyl;
R⁴ represents nitro, cyano, C₁₋₆alkoxy, C(=X)NR⁶R⁷, NR⁸R⁹, (CH₂)ₘNR¹⁰C(=Y)R¹¹ or a 5-membered heterocyclic ring selected from thienyl, thiazolyl and 1,2,4-triazolyl each ring optionally substituted by a C₁₋₄alkyl or aryl group; or when R¹ is arylmethyl or C₁₋₆alkyl substituted by one or more fluorine atoms then R⁴ may also represent hydrogen;
R⁵ represents hydrogen or C₁₋₆alkyl;
R⁶ represents hydrogen or C₁₋₆alkyl;
R⁷ represents hydrogen, amino, hydroxyl, C₁₋₆alkyl, aryl or arylC₁₋₄alkyl;
R⁸ represents hydrogen or C₁₋₆alkyl;
R⁹ represents hydrogen, C₁₋₆alkyl, SO₂R¹², CO₂R¹², C(=NCN)SR¹² or C(=NCN)NR¹³R¹⁴;
R¹⁰ represents hydrogen or C₁₋₆alkyl;
R¹¹ represents C₁₋₆alkyl optionally substituted by one or more halogen atoms, or R¹¹ represents aryl, arylC₁₋₄alkyl, thienyl, NR¹⁵R¹⁶, CH₂NR¹⁷R¹⁸ or R¹⁰ and R¹¹ together represent -A(CH₂)ₙ-;
R¹² represents C₁₋₆alkyl, aryl or arylC₁₋₄alkyl;
R¹³ represents hydrogen or C₁₋₆alkyl;
R¹⁴ represents hydrogen, C₁₋₆alkyl, aryl, arylC₁₋₄alkyl or R¹³ and R¹⁴ together with the nitrogen atom to which they are attached form a morpholine, piperazine or N-C₁₋₄alkylpiperazine ring;
R¹⁵ represents hydrogen or C₁₋₆alkyl or R¹⁰ and R¹⁵ together represent -A(CH₂)ₙ-;
R¹⁶ represents hydrogen, C₁₋₆alkyl, aryl, arylC₁₋₄alkyl, CO₂R¹², CH₂CO₂R¹² or R¹⁵ and R¹⁶ together with the nitrogen atom to which they are attached form a morpholine, piperazine or N-C₁₋₄alkylpiperazine ring;
R¹⁷ represents hydrogen or C₁₋₆alkyl;
R¹⁸ represents hydrogen, C₁₋₆alkyl, aryl, arylC₁₋₄alkyl, COR¹² or R¹⁷ and R¹⁸ together with the nitrogen atom to which they are attached form a morpholine, piperazine or N-C₁₋₄alkylpiperazine ring;
A represents CH₂ or C=O;
m represents zero or 1;
n represents 1,2 or 3;
X represents S or NH, or when R⁷ represents amino then X may also represent O;
Y represents O or S; for use in therapy.

In the above definition, unless otherwise indicated, alkyl and alkoxy groups having three or more carbon atoms may be straight chain or branched chain. The term 'aryl' as a group or part of a group means 2, 3 or 4-pyridyl, phenyl or phenyl substituted by one or more halogen atoms or C₁₋₄alkyl or C₁₋₄alkoxy groups, including compounds in which two alkoxy groups at adjacent positions on the phenyl ring are linked together to form an alkylidenedioxy group. The terms 'halogen' as used herein means fluorine, chlorine, bromide or iodine.

The compounds of formula (I) may contain one or more asymmetric centres and thus can exist as enantiomers or diastereoisomers. It is to be understood that the invention includes both mixtures and separate individual isomers of the compounds of formula (I).

The compounds of formula (I) may also exist in tautomeric forms and the invention includes both mixtures and separate individual tautomers thereof.

The pharmaceutically acceptable salts of the compounds of formula (I) which contain a basic centre are acid addition salts formed with pharmaceutically acceptable acids. Examples include the hydrochloride, hydrobromide, sulphate or bisulphate, phosphate or hydrogen phosphate, acetate, benzoate, succinate, fumarate, maleate, lactate, citrate, tartrate, gluconate, methanesulphonate, benzenesulphonate and p-toluenesulphonate salts. Compounds of the formula (I) can also provide pharmaceutically acceptable metal salts, in particular alkali metal salts, with bases. Examples include the sodium and potassium salts.

In a further aspect, the present invention provides compounds of formula (I) and salts and solvates (e.g. hydrates) thereof as defined hereinabove, with the proviso that when R¹ represents C₁₋₆alkyl and R⁵ represents hydrogen then R⁴ cannot represent nitro or NH₂.

Another aspect of the invention comprises compounds of formula (I) and salts and solvates (e.g. hydrates) thereof in which:
R¹ represents C₁₋₆alkyl, 2,2,2-trifluoroethyl or benzyl;
R² represents methyl;
R³ represents C₂₋₄alkyl;
R⁴ represents nitro, cyano, C₁₋₆alkoxy, C(=X)NR⁶R⁷, NR⁸R⁹, (CH₂)mNR¹⁰C(=Y)R¹¹ or thiazolyl or 1,2,4-triazolyl each ring optionally substituted by a C₁₋₄alkyl or aryl group;
R⁵ represents hydrogen or C₁₋₆alkyl;
R⁶ represents hydrogen or C₁₋₆alkyl;
R⁷ represents, hydrogen, amino, hydroxyl, C₁₋₆alkyl, aryl or arylC₁₋₄alkyl;
R⁸ represents hydrogen or C₁₋₆alkyl;
R⁹ represents hydrogen, C₁₋₆alkyl, SO₂R¹², CO₂R¹², C(=NCN)SR¹² or C(=NCN)NR¹³R¹⁴;
R¹⁰ represents hydrogen or C₁₋₆alkyl;
R¹¹ represents C₁₋₆alkyl optionally substituted by one or more halogen atoms, or R¹¹ represents aryl, arylC₁₋₄alkyl, thienyl, NR¹⁵R¹⁶, CH₂NR¹⁷R¹⁸ or R¹⁰ and R¹¹ together represent -A(CH₂)ₙ-;
R¹² represents C₁₋₆alkyl, aryl or arylC₁₋₄alkyl;
R¹³ represents hydrogen or C₁₋₆alkyl;
R¹⁴ represents hydrogen, C₁₋₆alkyl, aryl, arylC₁₋₄alkyl or R¹³ and R¹⁴ together with the nitrogen atom to which they are attached form a morpholine, piperazine or N-C₁₋₄alkylpiperazine ring;
R¹⁵ represents hydrogen or C₁₋₆alkyl or R¹⁰ and R¹⁵ together represent -A(CH₂)ₙ-;
R¹⁶ represents hydrogen, C₁₋₆alkyl, aryl, arylC₁₋₄alkyl, CO₂R¹², CH₂CO₂R¹² or R¹⁵ and R¹⁶ together with the nitrogen atom to which they are attached form a morpholine, piperazine or N-C₁₋₄alkylpiperazine ring;
R¹⁷ represents hydrogen or C₁₋₆alkyl;
R¹⁸ represents hydrogen, C₁₋₆alkyl, aryl, arylC₁₋₄alkyl, COR¹² or R¹⁷ and R¹⁸ together with the nitrogen atom to which they are attached form a morpholine, piperazine or N-C₁₋₄alkylpiperazine ring;
A represents CH₂ or C=O;
m represents zero or 1;
n represents 1,2 or 3;
X represents S or NH, or when R⁷ represents amino then X may also represent O;
Y represents O or S, with the proviso that when R¹ represents C₁₋₆alkyl and R⁵ represents hydrogen then R⁴ cannot represent nitro or NH₂.

A particular group of compounds of formula (I) are those in which R¹ represents a C₁₋₆alkyl group. R¹ preferably represents a C₁₋₃alkyl group, especially methyl or ethyl.

R³ preferably represents a C₂₋₃alkyl group, especially n-propyl.

Examples of the group R⁴ include hydrogen, nitro, cyano, C₁₋₄alkoxy (e.g. n-propoxy), C(=S)NHR⁷, C(=NH)NHR⁷, NHR⁹ [where R⁹ is hydrogen, SO₂C₁₋₄alkyl (e.g. SO₂CH₃), CO₂C₁₋₄alkyl (e.g. CO₂CH₃ or CO₂Et), C(=NCN)SC₁₋₄alkyl (e.g. C(=NCN)SCH₃) or C(=NCN)NHR¹⁴ where R¹⁴ is C₁₋₄alkyl (e.g. methyl or n-butyl)], NR⁸R⁹ [where R⁸ and R⁹ each independently represent C₁₋₄alkyl (e.g. methyl)], NHCOR¹¹ [where R¹¹ is C₁₋ ₄alkyl (e.g. methyl), aryl (e.g. p-fluorophenyl), thienyl (e.g. 2-thienyl), CH₂NR¹⁷R¹⁸, halomethyl (e.g. bromomethyl) or trifluoromethyl], thiazolyl (e.g. 2-thiazolyl or 4-thiazolyl) optionally substituted by methyl or aryl, 1,2,4-triazolyl optionally substituted by methyl or aryl, 2-thienyl optionally substituted by methyl or aryl, -NHC(=Y)NHR¹⁶, -NHC(=Y)CH₂NHCOR¹², (CH₂)ₘNR¹⁰C(=Y)R¹¹ where R¹⁰ and R¹¹ together represent -A(CH₂)ₙ- or (CH₂)ₘNR¹⁰C(=Y)NR¹⁵R¹⁶ where R¹⁰ and R¹¹ together represent -A(CH₂)ₙ-.

R⁴ preferably represents either (i) any group previously described which is linked to the benzene ring of the rest of the molecule via a nitrogen atom, for example NHCOR¹¹ (where R¹¹ is methyl, p-fluorophenyl, 2-thienyl or trifluoromethyl), NH₂, NHSO₂CH₃, NHCO₂CH₃, NHCO₂Et, NHCONHEt and NHCSNHEt or (ii) a thienyl, thiazolyl or 1,2,4-triazolyl ring each substituted by methyl or aryl.

When R⁵ is a C₁₋₆alkyl group it preferably represents a C₁₋₄alkyl group (e.g. methyl).

It is to be understood that the present invention covers all appropriate combinations of particular and preferred groupings hereinabove.

A preferred group of compounds of formula (I) for use according to the present invention are compounds of formula (Ia) 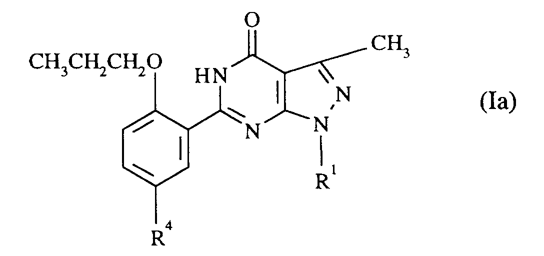
and physiologically acceptable salts and solvates (e.g. hydrates) thereof, in which R¹ represents C₁₋₃alkyl, especially methyl or ethyl, and R⁴ is as defined previously.

Particularly preferred individual compounds of formula (I) for use according to the invention include:
1,3-dimethyl-6-(2-propoxy-5-acetamidophenyl)-1,5-dihydropyrazolo[3,4-d]pyrimidin-4-one;
1-ethyl-3-methyl-6-[2-propoxy-5-(4-methyl-2-thiazolyl)phenyl]-1,5-dihydropyrazolo[3,4-d]pyrimidin-4-one;
1-ethyl-3-methyl-6-[2-propoxy-5-(2-methyl-4-thiazolyl)phenyl]-1,5-dihydropyrazolo[3,4-d]pyrimidin-4-one;
1-ethyl-3-methyl-6-[2-propoxy-5-(2-(3-pyridyl)-4-thiazolyl)phenyl]-1,5-dihydropyrazolo[3,4-d]pyrimidin-4-one;
1,3-dimethyl-6-[2-propoxy-5-(2-methyl-4-thiazolyl)phenyl]-1,5-dihydropyrazolo[3,4-d]pyrimidin-4-one;
1,3-dimethyl-6-[2-propoxy-5-(3-phenyl-1,2,4-triazol-5-yl)phenyl]-1,5-dihydropyrazolo[3,4-d]pyrimidin-4-one;
1,3-dimethyl-6-(2-propoxy-5-methanesulfonamidophenyl)-1,5-dihydropyrazolo[3,4-d]pyrimidin-4-one; and
1,3-dimethyl-6-(2-propoxy-5-aminophenyl)-1,5-dihydropyrazolo[3,4-d]pyrimidin-4-one; and physiologically acceptable salts and solvates (e.g. hydrates) thereof.

Other preferred individual compounds of formula (I) for use according to the invention include:
1,3-dimethyl-6-[2-propoxy-5-(4-trifluoroacetamido)phenyl]-1,5-dihydropyrazolo[3,4-d]pyrimidin-4-one;
1,3-dimethyl-6-[2-propoxy-5-(4-fluorobenzamido)phenyl]-1,5-dihydropyrazolo[3,4-d]pyrimidin-4-one;
1,3-dimethyl-6-[2-propoxy-5-((2-thienyl)carboxamido)phenyl]-1-5-dihydropyrazolo[3,4-d]pyrimidin-4-one;
1,3-dimethyl-6-(2-propoxy-5-ethoxycarbonylaminophenyl)-1,5-dihydropyrazolo[3,4-d]pyrimidin-4-one;
1,3-dimethyl-6-(2-propoxy-5-ethylureidophenyl)-1,5-dihydropyrazolo[3,4-d]pyrimidin-4-one;
1,3-dimethyl-6-(2-propoxy-5-ethylthioureidophenyl)-1,5-dihydropyrazolo[3,4-d]pyrimidin-4-one; and
1,3-dimethyl-6-[2-propoxy-5-(5-methyl-2-thienyl)phenyl]-1,5-dihydropyrazolo[3,4-d]pyrimidin-4-one;
and physiologically acceptable salts and solvates (e.g. hydrates) thereof.

The affinities of compounds of formula (I) for cGMP specific PDE (Phosphodiesterase type V) may be assessed by determination of their IC₅₀ values (the concentration of inhibitor required for 50% inhibition of enzyme activity). The PDE V in enzyme is isolated from bovine aorta, essentially by the method of Lugnier et al. in Biochem. Pharmacology 35, 1743 (1986) and assays are performed using a "one step" assay adapted from the method of Wells et al in Biochim. Biophys. Acta 384, 430 (1975). Results of these tests show that compounds of the present invention are potent inhibitors of cGMP specific PDE. Tests against other PDE enzymes using standard methodology also show that compounds of the invention are highly selective for the cGMP specific PDE enzyme.

Thus, as indicated above, compounds of formula (I) are of interest for use in therapy, specifically for the treatment of a variety of conditions where inhibition of cGMP specific PDE is thought to be beneficial.

As a consequence of the selective PDE V in inhibition exhibited by compounds of the present invention, cGMP levels are elevated, which in turn can give rise to beneficial anti-platelet, anti-neutrophil, anti-vasospastic, vasodilatory, natriuretic and diuretic activities as well as potentiation of the effects of endothelium-derived relaxing factor (EDRF), nitrovasodilators, atrial natriuretic factor (ANF) and endothelium-dependent relaxing agents such as bradykinin, acetylcholine and 5-HT₁. The compounds of formula (I) therefore have utility in the treatment of a number of disorders, including stable, unstable and variant (Prinzmetal) angina, hypertension, pulmonary hypertension, congestive heart failure, renal failure, atherosclerosis, conditions of reduced blood vessel patency (e.g. post-percutaneous transluminal coronary angioplasty), peripheral vascular disease, stroke, bronchitis, chronic asthma, allergic asthma, allergic rhinitis, glaucoma and diseases characterised by disorders of gut motility (e.g. irritable bowel syndrome).

It will be appreciated that references herein to treatment extend to prophylaxis as well as treatment of established conditions.

It will also be appreciated that references herein to 'a compound of formula (I)' embrace such a compound or a physiologically acceptable salt or solvate thereof, or a pharmaceutical composition containing either entity.

There is thus provided as a further aspect of the invention a compound of formula (I) for use in the treatment of stable, unstable and variant (Prinzmetal) angina, hypertension, pulmonary hypertension, congestive heart failure, renal failure, atherosclerosis, conditions of reduced blood vessel patency, (e.g. post-PTCA), peripheral vascular disease, stroke, bronchitis, chronic asthma, allergic asthma, allergic rhinitis, glaucoma or diseases characterised by disorders of gut motility (e.g. IBS).

According to another aspect of the invention, there is provided the use of a compound of formula (I) for the manufacture of a medicament for the treatment of stable, unstable and variant (Prinzmetal) angina, hypertension, pulmonary hypertension, congestive heart failure, renal failure, atherosclerosis, conditions of reduced blood vessel patency, (e.g. post-PTCA), peripheral vascular disease, stroke, bronchitis, chronic asthma, allergic asthma, allergic rhinitis, glaucoma or diseases characterised by disorders of gut motility (e.g. IBS).

In a further aspect, the invention provides a method of treating stable, unstable and variant (Prinzmetal) angina, hypertension, pulmonary hypertension, congestive heart failure, renal failure, atherosclerosis, conditions of reduced blood vessel patency, (e.g. post-PTCA), peripheral vascular disease, stroke, bronchitis, chronic asthma, allergic asthma, allergic rhinitis, glaucoma or diseases characterised by disorders of gut motility (e.g. IBS) in a human or non-human animal body which comprises administering to said body a therapeutically effective amount of a compound with formula (I).

For administration to man in the curative or prophylactic treatment of the disorders identified above, oral dosages of a compound of formula (I) will generally be in the range of from 4-800mg daily for an average adult patient (70kg). Thus for a typical adult patient, individual tablets or capsules contain from 2-400mg of active compound, in a suitable pharmaceutically acceptable vehicle or carrier, for administration in single or multiple doses, once or several times per day. Dosages for intravenous, buccal or sublingual administration will typically be within the range of from 1-400 mg per single dose as required. In practice the physician will determine the actual dosing regimen which will be most suitable for an individual patient and it will vary with the age, weight and response of the particular patient. The above dosages are exemplary of the average case but there can be individual instances in which higher or lower dosage ranges may be merited, and such are within the scope of this invention.

For human use, a compound of the formula (I) can be administered alone, but will generally be administered in admixture with a pharmaceutical carrier selected with regard to the intended route of administration and standard pharmaceutical practice. For example, the compound may be administered orally, buccally or sublingually, in the form of tablets containing excipients such as starch or lactose, or in capsules or ovules either alone or in admixture with excipients, or in the form of elixirs or suspensions containing flavouring or colouring agents. A compound may also be injected parenterally, for example intravenously, intramuscularly, subcutaneously or intracoronarily. For parenteral administration, the compound is best used in the form of a sterile aqueous solution which may contain other substances, for example salts, or monosaccharides such as mannitol or glucose, to make the solution isotonic with blood.

Thus, the invention provides in a further aspect a pharmaceutical composition comprising a compound of the formula (I) together with a pharmaceutically acceptable diluent or carrier.

A compound of formula (I) may also be used in combination with other therapeutic agents which may be useful in the treatment of the above-mentioned disease states. The invention thus provides, in another aspect, a combination of a compound of formula (I) together with another therapeutically active agent.

The combination referred to above may conveniently be presented for use in the form of a pharmaceutical formulation and thus pharmaceutical compositions comprising a combination as defined above together with a pharmaceutically acceptable diluent or carrier comprise a further aspect of the invention.

The individual components of such a combination may also be administered either sequentially or simultaneously in separate pharmaceutical formulations.

Appropriate doses of known therapeutic agents for use in combination with a compound of formula (I) will be readily appreciated by those skilled in the art.

Compounds of formula (I) may be prepared by any suitable method known in the art or by the following processes which form part of the present invention. In the methods below R¹ to R⁵ are as defined in formula (I) above unless otherwise indicated.

Thus, a first process (A) for preparing a compound of formula (I) in which R⁴ represents nitro or alkoxy comprises cyclising a compound of formula (II) 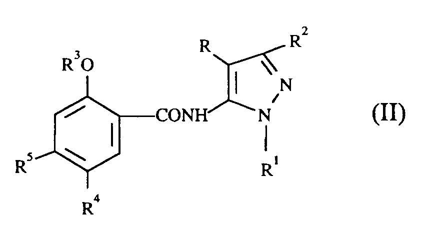
(in which R⁴ is nitro or C₁₋₆alkoxy and R is a group CN or CONH₂). Thus, for example, when R is CN the reaction may be effected by treating (II) with a strong oxidising agent such as a peroxide oxidising agent (e.g. hydrogen peroxide) in the presence of a suitable base such as an alkali earth metal hydroxide (e.g. sodium hydroxide), conveniently at an elevated temperature (e.g. 50° to 120°C). When R is CONH₂ the reaction may be effected by treat (II) with a suitable base such as an alkali earth metal hydroxide (e.g. sodium hydroxide), conveniently at an elevated temperature (e.g. 50⁰ to 120⁰C) and optionally also comprising a solvent such as an alcohol (e.g. ethanol).

In another process (B), compounds of formula (I) may be prepared from other compounds of formula (I) by interconversion reactions.

Thus, in one embodiment of process (B), a compound of formula (I) in which R⁴ represents NH₂ may be prepared from a corresponding compound of formula (I) in which R⁴ represents NO₂ using conventional reducing conditions such as hydrogenation in the presence of a palladium catalyst (e.g. palladium-on-carbon).

In a further embodiment of process (B), a compound of formula (I) in which R⁴ represents NO₂ may be prepared from a corresponding compound of formula (I) in which R⁴ is hydrogen under conventional nitration conditions, for example using a concentrated nitric acid/concentrated sulfuric acid combination.

In another embodiment of process (B), a compound of formula (I) in which R⁴ represents NH₂ may be converted to a corresponding compound of formula (I) in which R⁴ represents NR⁸R⁹ or NR⁸COR¹⁰ using conventional methodology. Examples of appropriate N-substitution reactions are provided in the Examples Section hereinafter. These include N-sulfonylation which may be effected using a sulfonyl halide (e.g. a sulfonyl chloride) in the presence of a base such as triethylamine and in an organic solvent (e.g. an ether such as tetrahydrofuran); N-acylation using an acid chloride under similar conditions to those referred to above for the N-sulfonylation reaction; and N-aminocarbonylation by reacting the amine with a reagent R¹⁵N=C=Y, optionally in the presence of an organic acid (e.g. acetic acid). N-methylation may be achieved, for example, using formaldehyde and formic acid, conveniently at an elevated temperature (e.g. about 80°C).

In another embodiment of process (B), a compound of formula (I) in which R⁴ contains a 2-thiazolyl group may conveniently be prepared from the corresponding cyano compound via a compound of formula (I) in which R⁴ is C(=S)NH₂. The transformations may be effected by conventional sulfuration and ring closure procedures, for examples as described in the Examples Section hereinafter.

In a further embodiment of process (B), a compound of formula (I) in which R⁴ contains a 1,2,4-triazolyl group may be prepared by treating the corresponding compound of formula (I) in which R⁴ is CONHNH₂ with a thiocarboxylic acid amide in a hydrocarbon solvent (e.g. xylene) at an elevated temperature (e.g. reflux).

According to another process (C), a compound of formula (I) may be prepared by reacting a compound of formula (I) in which R⁴ is a reactive atom or group to introduce the desired R⁴ group.

Thus, in one embodiment of process (C) a compound of formula (I) in which R⁴ is CONHNH₂ may be prepared by treating a corresponding carboxylic acid ester (known from WO93/07149) with hydrazine hydrate in an alcoholic solvent at an elevated temperature (e.g. reflux).

According to a further embodiment of process (C), a compound of formula (I) in which R⁴ contains a 4-thiazolyl group may conveniently be prepared from a corresponding compound of formula (I) in which R⁴ is a hydrogen atom via Friedel-Crafts chemistry as described in WO93/07149 using a haloacetyl halide to provide a halomethylketone and reacting said halomethylketone with a thioamide at an elevated temperature (e.g. reflux) in a suitable solvent such as an alcohol (e.g. ethanol).

In a further embodiment of process (C), compounds of formula (I) may be prepared from corresponding compounds of formula (I) in which R⁴ is a halogen (e.g. bromine atom) or a halomethyl (e.g. bromomethyl) group, which may in turn be prepared according to the procedures described in WO93/07149. Thus, compounds of formula (I) in which R⁴ is cyano may be prepared by treating the corresponding bromo compound with an alkali metal cyanide using conventional conditions, for example as described in the Examples Section hereinafter. The halomethyl compounds may be converted to corresponding compounds in which R⁴ represents a group CH₂NR¹⁰C(=Y)R¹¹ by a conventional displacement reaction conveniently in the presence of a strong base such as sodium hydride in dimethylformamide. Compounds of formula (I) in which R⁴ is an optionally substituted thienyl ring may be prepared by treating the corresponding compound in which R⁴ is bromine with a trialkyltinthiophene compound such as a trimethyltinthiophene compound in the presence of a palladium catalyst (e.g. bis(triphenylphosphine)palladium (II) chloride) in a suitable solvent such as an ether solvent (e.g. tetrahydrofuran) at an elevated temperature (e.g. reflux).

Compounds of formula (I) in which R⁴ is halomethyl may also be prepared from corresponding compounds of formula (I) in which R⁴ is hydrogen by a conventional sequence of reaction steps. Thus, as a first step, the unsubstituted compound may be converted to a corresponding compound in which R⁴ is methylketone, which may then be made to form a carboxyl group by reaction with bromine and sodium hydroxide. This is then reduced to a hydroxymethyl group which is converted by halogenation to the desired halomethyl group.

Intermediate compounds of formula (II) may conveniently be prepared by the coupling of compounds of formulae (III) and (IV). 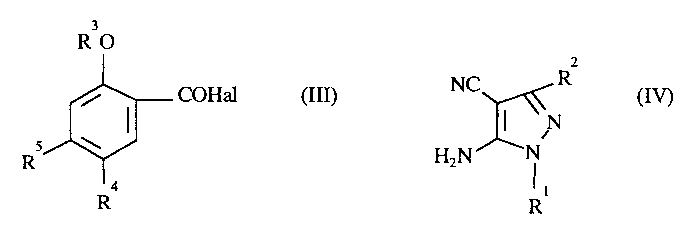
(where Hal is a halogen atom, e.g. chlorine) using procedures essentially as described in WO93/07149.

It will be appreciated that R⁴ may be converted to a different R⁴ grouping as an intermediate step in the overall synthesis of compounds of formula (I) whereas process (B) hereinabove describes interconversion as a final step process.

Compounds of formula (II) are novel intermediates and represent a further aspect of the present invention.

Compounds of formulae (III) and (IV) are either known in the art, for example as described in WO93/07149, or may be prepared by methods analogous to methods described in the art for preparing known compound of formulae (III) and (IV), and by methods described in the Examples Section hereinafter.

The pharmaceutically acceptable acid addition salts of the compounds of formula (I) which contain a basic centre may be prepared in a conventional manner. For example, a solution of the free base may be treated with a suitable acid, either neat or in a suitable solution, and the resulting salt isolated either by filtration or by evaporation under vacuum of the reaction solvent. Pharmaceutically acceptable base addition salts may be obtained in an analogous manner by treating a solution of a compound of formula (I) with a suitable base. Both types of salt may be formed or interconverted using ion-exchange resin techniques.

The synthesis of the compounds of the invention and of the intermediates for use therein are illustrated by the following, non-limiting Examples. In the following Examples DMSO means dimethylsulphoxide and DMF means N,N-dimethylformamide.

### INTERMEDIATE 1

### 5-Amino-4-cyano-1,3-dimethylpyrazole

Methylhydrazine (8.5 g, 0.185 mol) was added dropwise to a stirred solution of (1-ethoxyethylidene)malononitrile (25 g, 0.184 mol) in ethanol (100 ml) at room temperature. The mixture was then heated 2 hours under reflux and cooled. The crystals were filtrated, washed with ethanol, ether and dried to give the title compound (21 g) m.p. : 193°C.

### INTERMEDIATE 2

### 5-Amino-4-cyano-1-ethyl-3-methylpyrazole

A suspension of ethylhydrazine oxalate (25 g, 0.166 mol) in methanol (1 litre) was treated at reflux with sodium methoxide (17 g, 0.34 mol) during 15 minutes. The mixture was filtered, (1-ethoxyethylidene)malononitrile (18.9g, 0.139 mol) was added into the methanolic solution and the reflux was continued 2 hours. The solution was then concentrated in vacuo and the residue extracted with diethyl ether. The ethereal solution was concentrated to give the title compound as off white crystals (19 g) m.p: 120-2°C.

### INTERMEDIATE 3

### 4-Cyano-1,3-dimethyl-5-[di(2-propoxy-5-nitrobenzoyl)amino]pyrazole

2-propoxy-5-nitrobenzoyl chloride (48.7 g, 0.2 mol) was added dropwise to a solution of Intermediate 1 (13.6 g, 0.1 mol) in 250 ml of pyridine at room temperature. After stirring 3 hours at 50°C, the mixture was poured into cold water. The precipitate was filtered off, washed with water, dried and recrystallized from acetonitrile to give the title compound as cream crystals (50 g) m.p : 219-20°C.

| *Analysis* : C₂₆H₂₆N₆O₈ | | | |
|---|---|---|---|
| *Calculated* : | C, 56.71 ; | H, 4.76 ; | N, 15.26 |
| *Found* : | C, 56.90 ; | H, 4.91 ; | N, 15.18 |

### INTERMEDIATE 4

### 5-Amino-4-carboxamido-1,3-dimethylpyrazole

Intermediate 1 (10 g) was added slowly to sulfuric acid (50 ml) while maintaining the temperature below 10°C with stirring. The mixture was then heated 2 hours at 60°C and poured into crushed ice. The solution was made slightly basic (pH 9) with 20% ammonium hydroxide solution and the resulting crystals were filtered to give the title compound as off white crystals (9 g) m.p. : 202°C.

### INTERMEDIATE 5

### 1,3-Dimethyl-6-(5-bromo-2-propoxyphenyl)-1,5-dihydropyrazolo[3,4-d]pyrimidin-4-one

Bromine (0.3 ml, 5.5 mmol) was added dropwise to a solution of 1,3-dimethyl-6-[2-propoxyphenyl]-1,5-dihydro-pyrazolo[3,4-d]pyrimidin-4-one (prepared according to the method described in WO93/07149; 1 g, 3.3 mmol) in acetic acid (20 ml) and the mixture was heated at 100°C for 5 hours, and then poured into water. The product was extracted with dichloromethane, the organic phase was dried (Na₂SO₄) and concentrated to give a solid. After crystallisation from isopropanol, the title compound was obtained as white crystals (0.43 g) m.p. : 135°C.

| *Analysis* : C₁₆H₁₇BrN₄O₂ | | | |
|---|---|---|---|
| *Calculated* : | C, 50.94 ; | H, 4.54 ; | N, 14.85 |
| *Found* : | C, 51.86 ; | H, 4.93 ; | N, 14.22 |

### INTERMEDIATE 6

### 1,3-Dimethyl-6-(2-isopropoxyphenyl)-1,5-dihydro-pyrazolo[3,4-d]pyrimidin-4-one

The same method as used in the preparation of Example 2, but starting from 2-isopropoxybenzoyl chloride and Intermediate 1, gave, after crystallization from water/dimethylformamide, the title compound as light yellow crystals (0.76 g) m.p. : 128°C.

| *Analysis* : C₁₆H₁₈N₄O₂ | | | |
|---|---|---|---|
| *Calculated* : | C, 64.41 ; | H, 6.08 ; | N, 18.78 |
| *Found* : | C, 64.49 ; | H, 6.17 ; | N, 18.86 |

### INTERMEDIATE 7

### 1,3-Dimethyl-6-(4-methyl-2-propoxyphenyl)-1,5-dihydro-pyrazolo[3,4-d]pyrimidin-4-one

The same method as used in the preparation of Example 2, but starting from 4-methyl-2-propoxybenzoyl chloride and Intermediate 1, gave, after crystallization from methanol, the title compound as yellow crystals (1.7 g) m.p. : 190°C.

| *Analysis* : C₁₇H₂₀N₄O₂ | | | |
|---|---|---|---|
| *Calculated* : | C, 65.37 ; | H, 6.45 ; | N, 17.94 |
| *Found* : | C, 64.79 ; | H, 6.42 ; | N, 17.78 |

### INTERMEDIATE 8

### 5-Amino-4-cyano-1-(2,2,2 trifluoroethyl)-3-methylpyrazole

2,2,2-Trifluoroethylhydrazine [70% in water (25g)] was added to a solution of (1-ethoxyethylidene)malononitrile (20g) in ethanol (200 ml) at room temperature. The mixture was heated 2 hours under reflux and concentrated. The resulting oil solidified and the crystals were washed with ether to give the title compound (25 g) m.p. : 132°C.

### INTERMEDIATE 9

### 5-Amino-4-cyano-3-methylpyrazole

Hydrazine monohydrate (17.1ml) was added dropwise to a stirred solution of (1-ethoxyethylidene)malonitrile (40g) in ethanol (500 ml) at room temperature. The mixture was heated 1 hour under reflux and then concentrated under reduced pressure. The residue, by trituration with diethyl ether, crystallised. The crystals were filtrated and dried to give the title compound as light pink crystals (31.6 g) mp : 166°C.

### INTERMEDIATE 10

### 5-Amino-1-benzyl-4-cyano-3-methylpyrazole

A suspension of benzylhydrazine hydrochloride (25g) in ethanol (800 ml) was treated at reflux with sodium methoxide (13.8g) during 15 minutes and (1-ethoxyethylidene)malonitrile (14.5g) was added portionwise. The reflux was continued 3 hours. The solution was concentrated to about 200 ml and the product crystallised by addition of diisopropylether. The crystals were filtrated and dried to give the title compound as light brown crystals (17 g) mp : 114-116°C.

### INTERMEDIATE 11

### 5-Amino-4-cyano-3-methyl-1-(3-pyridylmethyl)pyrazole

A solution of Intermediate 9 (5g) in DMF (50 ml) was added dropwise to a stirred suspension of sodium hydride (60 % in mineral oil ; 1.72g) in DMF (50 ml) and the mixture was stirred at room temperature during 15 mn. A solution of 3-chloromethylpyridine (6.27g) in DMF (60 ml) was added dropwise and the mixture was heated at 60°C during 3 hours and then concentrated under reduced pressure. The residue was diluted with water, extracted with dichloromethane. The organic phase was dried (Na₂SO₄) and concentrated in vacuo. The two isomers were separated by column chromatography on silica gel, eluting with dichloromethane/methanol (95/5 then 9/1). The title compound was obtained as an orange solid (2.57g).
NMR (DMSO, 250 MHz, ppm) : 1.95 (s, 3H, CH₃) ; 5.05 (s, 2H, CH₂ py) ; 6.6 (s, 2H, NH₂) ; 7.2 (dd, 1H, H₅py) ; 7.4 (d, 1H, H₆ py) ; 8.3 (s, 1H, H₂ py) ; 8.35 (d, 1H, H₄ py)

### INTERMEDIATE 12

### 5-Amino-4-cyano-3-methyl-1-(4-pyridylmethyl)pyrazole

The same method as used in the preparation of Intermediate 11 but starting from 4-chloromethylpyridine, gave the title compound as a solid.
NMR (DMSO, 250 MHz, ppm) : 2.05 (s, 3H, CH₃) ; 5.15 (s,2H, CH₂py) ; 6.7 (s, 2H, NH₂) ; 7.05 (d, 2H, H2 + H₆py) ; 8.5 (d, 2H, H₃ + H₅py)

### INTERMEDIATE 13

### 5-Amino-4-cyano-3-methyl-1-(2-pyridylmethyl)pyrazole

The same method as used in the preparation of Intermediate 11 but starting from 2-chloromethylpyridine, gave the title compound as light brown solid.
NMR (DMSO, 250 MHz, ppm) : 2.0 (s 3H, CH₃) ; 5.1 (s, 2H, CH₂py) ; 6.6 (s, 2H, NH₂) ; 6.95 (d, 1H, H₆py) ; 7.25 (t, 1H, H₅py) ; 7.75 (t, 1H, H₄py) ; 8.5 (d, 1H, H₃py)

### INTERMEDIATE 14

### 5-Amino-4-cyano-3-methyl-1-(3,4-methylenedioxybenzyl)pyrazole

The same method as used in the preparation of Intermediate 11 but starting from 3,4-methylenedioxybenzylbromide, gave the title compound as a light brown solid.
NMR (DMSO, 260 MHz, ppm) : 2.05 (s, 3H, CH₃) ; 5.0 (s, 2H, CH₂) ; 6.05 (s, 2H, O-CH₂-O); 6.7 (s, 2H, NH₂) ; 6.75 (dd, 1H, H₆Ar) ; 6.85 (sd, 1H, H₂Ar) ; 6.95 (d, 1H, H₅ Ar)

### INTERMEDIATE 15

### 1,3-Dimethyl-6-[2-propoxy-5-(hydroxymethyl)phenyl]-1,5-dihydropyrazolo[3,4-d]pyrimidin-4-one

A solution of lithium aluminium hydride in tetrahydrofuran (1M, 13.4 ml) was added dropwise to a solution of 1,3-dimethyl-6-(2-propoxy-5-methylcarboxyphenyl-1,5-dihydro-pyrazolo[3,4-d]pyrimidin-4-one (prepared according to the method described in WO93/07149; 2.3 g; 6.7 mmol) in tetrahydrofuran (100 ml) and the mixture was heated under reflux during 1 hour. After cooling, water (30 ml) was added dropwise and the mixture was concentrated under reduced pressure. The residue was treated with water and extracted with dichloromethane. The organic layer was dried (Na₂SO₄) and concentrated. The residue was crystallised from isopropanol to afford the title compound as yellow crystals (1.04 g) mp : 196°C.

### INTERMEDIATE 16

### 1,3-Dimethyl-6-[2-propoxy-5-(bromomethyl)phenyl]-1,5-dihydropyrazolo[3,4-d]pyrimidin-4-one

Phosphorus tribromide (0.14 ml; 1.52 mmol) was added dropwise to a solution of Intermediate 15 (0.5 g, 1.52 mmol) in dichloromethane (20 ml) and the mixture was stirred at room temperature during 1 hour. After cooling, water (10 ml) was added dropwise, and the product extracted with dichloromethane. The organic layer was dried (Na₂SO₄) and concentrated under reduced pressure to leave a solid which was purified by column chromatography on silica gel eluting with dichloromethane/methanol (98/2). The title compound was obtained as white solid (0.5 g).
NMR (DMSO, 250 MHz, ppm) : 0.9 (t, 3H, CH₃ (OPr)); 1.6 (m, 2H, CH₂ (OPr)); 2.4 (s, 3H, CH₃); 3.8 (s, 3H, N-CH₃); 4.0 (t, 3H, O-CH₂); 4.7 (s, 2H, CH₂Br); 7.15 (d, 1H, H₃Ar); 7.6 (dd, 1H, H₄Ar); 7.8 (sd, 1H, H₆Ar); 11.75 (s, 1H, NH)

### EXAMPLE 1

### 1,3-Dimethyl-6-(2-propoxy-5-nitrophenyl)-1,5-dihydropyrazolo[3,4-d]pyrimidin-4-one

A solution of 30 % hydrogen peroxide (100 ml) was added to a stirred suspension of Intermediate 3 (50 g) in 0.5 N sodium hydroxide (1 litre). The mixture was then heated at 90°C during 18 hours to give a homogenous solution that was poured into water. The solution was made acidic with dilute hydrochloric acid and the precipitate was filtered off, washed with water and dried. After recrystallisation from ethanol the title compound was obtained as pale yellow crystals (20 g) m.p : 228-9°C.

| *Analysis* : C₁₆H₁₇N₅O₄ | | | |
|---|---|---|---|
| *Calculated* : | C, 55.96 ; | H, 4.99 ; | N, 20.39 |
| *Found* : | C, 56.19 ; | H, 4.94 ; | N, 20.04 |

### EXAMPLE 2

### 1-Ethyl-3-methyl-6-(2-propoxy 5-nitrophenyl)-1,5-dihydro-pyrazolo [3,4-d]pyrimidin-4-one

2-propoxy-5-nitrobenzoyl chloride (2.43 g) was added to a solution of Intermediate 2 (1.5 g) in pyridine (20 ml). The mixture was heated 3 hours at 60°C and poured into water. The resulting precipitate was filtrated off, washed with water and then with ethanol and dried. This crude product was heated at 100°C with stirring in a mixture of 1N sodium hydroxide (50 ml), water (30 ml) and 30 % hydrogen peroxide (3 ml) during 18 hours. The solution was filtrated and made acidic with dilute hydrochloric acid. The resulting precipitate was filtered off, washed with water and dried. After purification by chromatography on silica gel with a mixture of dichloromethane-methanol 95/5 as eluent and recrystallisation from ethanol, the title compound was obtained as pale yellow crystals (330 mg), m.p : 202-4°C.

| *Analysis* : C₁₇H₁₉N₅O₄ | | | |
|---|---|---|---|
| *Calculated* : | C, 57.13 ; | H, 6.36 ; | N, 19.60 |
| *Found* : | C, 56.85 ; | H, 5.34 ; | N, 20.07 |

The following compounds were obtained in a similar manner :

### EXAMPLE 3

### 1-Ethyl-3-methyl-6-(2-ethoxy-5-nitrophenyl)-1,5-dihydro-pyrazolo[3,4-d]pyrimidin-4-one

The same method, but starting from 2-ethoxy-5-nitrobenzoyl chloride and Intermediate 2 gave, after recrystallisation from ethanol, the title compound as white crystals mp : 224-6°C.

| *Analysis* : C₁₆H₁₇N₅O₄ | | | |
|---|---|---|---|
| *Calculated* : | C, 55.96 ; | H, 4.99 ; | N, 20.40 |
| *Found* : | C, 55.66 ; | H, 4.98 ; | N, 19.88 |

### EXAMPLE 4

### 1,3-Dimethyl-6-(2,5-dipropoxyphenyl)-1,5-dihydro-pyrazolo[3,4-d]pyrimidin-4-one

The same method, but starting from 2,5-dipropoxybenzoyl chloride and Intermediate 1 gave, after recrystallisation from isopropanol, the title compound as white crystals m.p. : 118°C.

| *Analysis* : C₁₉H₂₄N₄O₃ | | | |
|---|---|---|---|
| *Calculated* : | C, 64.03 ; | H, 6.79 ; | N, 15.72 |
| *Found* : | C, 64.15 ; | H, 6.75 ; | N, 15.80 |

### EXAMPLE 5

### 1-Ethyl-3-methyl-6-(2,5-dipropoxyphenyl)-1,5-dihydropyrazolo[3,4-d]pyrimidin-4-one

The same method, but starting from 2,5-dipropoxybenzoyl chloride and Intermediate 2 gave, after recrystallisation from diisopropyl ether, the title compound as white crystals m.p. : 108°C.

| *Analysis* : C₂₀H₂₆N₄O₃ | | | |
|---|---|---|---|
| *Calculated* : | C, 64.85 ; | H, 7.07 ; | N, 15.12 |
| *Found* : | C, 64.80 ; | H, 7.15 ; | N, 15.32 |

### EXAMPLE 6

### 1,3-Dimethyl-6-(2-ethoxy-5-nitrophenyl)-1,5-dihydropyrazolo[3,4-d]pyrimidin-4-one

2-Ethoxy-5-nitrobenzoyl chloride (2.3g, 10 mmol) was added to a solution of Intermediate 4 (1.54 g, 10 mmol) in pyridine (20 ml). The mixture was stirred overnight at room temperature and poured into water. The resulting precipitate was filtered off, washed with water, ethanol and dried. This product was taken up with 1N sodium hydroxide (50 ml) and ethanol (10 ml) and refluxed during 4 hours with stirring. The solution was then poured into water, acidified with dilute hydrochloric acid and the resulting precipitate was filtered off, washed with water and dried. Purification by chromatography on silica gel with a mixture of dichloromethane-methanol(95/5) afforded the title compound as white crystals (210 mg) m.p. : 280°C.

| *Analysis* : C₁₅H₁₅N₅O₄ | | | |
|---|---|---|---|
| *Calculated* : | C, 54.70 ; | H, 4.59 ; | N, 21.27 |
| *Found* : | C, 54.49 ; | H, 4.67 ; | N, 20.94 |

### EXAMPLE 7

### 1,3-Dimethyl-6-(2-propoxy-5-aminophenyl)-1,5-dihydropyrazolo[3,4-d]pyrimidin-4-one

Example 1 (7 g, 21 mmol)) in solution in tetrahydrofuran (250 ml) and ethanol (250 ml) was hydrogenated in presence of 10% Pd/C (0.7 g) at room temperature. After absorption of the theoretical volume of hydrogen, the mixture was filtered over a celite pad and concentrated in vacuo to give a yellow solid. Recrystallisation from acetonitrile gave the title compound as pale yellow crystals (6 g) m.p : 140°-142°C.

| *Analysis* : C₁₆H₁₉N₅O₂, 0,5H₂O | | | |
|---|---|---|---|
| *Calculated* : | C, 59.61 ; | H, 6.25 ; | N, 21.72 |
| *Found* : | C, 59.67 ; | H, 6.20 ; | N, 22.52 |

The following compounds were obtained in a similar manner :

### EXAMPLE 8

### 1-Ethyl-3-methyl-6-(2-propoxy-5-aminophenyl)-1,5-dihydro-pyrazolo[3,4-d]pyrimidin-4-one

The same method, but starting from Example 2, gave, after crystallisation from acetonitrile, the title compound as pale yellow crystals m.p : 167°C.

| *Analysis* : C₁₇H₂₁N₅O₂ | | | |
|---|---|---|---|
| *Calculated* : | C, 62.36 ; | H, 6.46 ; | N, 21.39 |
| *Found* : | C, 62.30 ; | H, 6.32 ; | N, 22.00 |

### EXAMPLE 9

### 1,3-Dimethyl-6-(2-ethoxy-5-aminophenyl)-1,5-dihydropyrazolo[3,4-d]pyrimidin-4-one

The same method, but starting from Example 6, gave, after crystallisation from ethanol, the title compound as yellow crystals m.p. : 208-10°C.

| *Analysis* : C₁₅H₁₇N₅O₂ | | | |
|---|---|---|---|
| *Calculated* : | C, 60.18 ; | H, 5.72 ; | N, 23.40 |
| *Found* : | C, 60.88 ; | H, 5.64 ; | N, 23.44 |

### EXAMPLE 10

### 1-Ethyl-3-methyl-6-(2-ethoxy-5-aminophenyl)-1,5-dihydropyrazolo[3,4-d]pyrimidin-4-one

The same method, but starting from Example 3, gave, after recrystallisation from ethanol, the title compound as yellow crystals m.p. : 195°C.

| *Analysis* : C₁₆H₁₉N₅O₂ | | | |
|---|---|---|---|
| *Calculated* : | C, 61.33 ; | H, 6.11 ; | N, 22.35 |
| *Found* : | C, 61.22 ; | H, 6.24 ; | N, 22.34 |

### EXAMPLE 11

### 1,3-Dimethyl-6-(2-propoxy-5-methanesulfonamidophenyl)-1,5-dihydropyrazolo[3,4-d]pyrimidin-4-one

Methanesulfonyl chloride (0.5 ml) was added to a solution of Example 7 (625 mg, 2 mmol) in tetrahydrofuran (50 ml) and triethylamine (0.6 ml) and the mixture was stirred 1 hour at room temperature. The mixture was then concentrated in vacuo treated with water and extracted with dichloromethane, the organic layer was washed with water, dried (Na₂SO₄) and concentrated. The residue was recrystallized from ethanol to afford the title compound as white crystals (300 mg) m.p : 235-237°C.

| *Analysis* : C₁₇H₂₁N₅O₄S | | | |
|---|---|---|---|
| *Calculated* : | C, 52.15 ; | H, 5.41 ; | N, 17.89 |
| *Found* : | C, 51.51 ; | H, 5.34 ; | N, 17.79 |

The following compounds were prepared in a similar manner :

### EXAMPLE 12

### 1-Ethyl-3-methyl-6-(2-propoxy-5-methanesulfonamidophenyl)-1,5-dihydropyrazolo[3,4-d] pyrimidin-4-one

The same method, but starting from Example 8, gave, after recrystallisation from ethanol, the title compound as white crystals m.p : 244-6°C.

| *Analysis* : C₁₈H₂₃N₅O₄S | | | |
|---|---|---|---|
| *Calculated* : | C, 53.31 ; | H, 5.72 ; | N, 17.23 |
| *Found* : | C, 53.12 ; | H, 5.74 ; | N, 17.21 |

### EXAMPLE 13

### 1,3-Dimethyl-6-(2-ethoxy-5-methanesulfonamidophenyl)-1,5-dihydropyrazolo[3,4-d]pyrimidin-4-one

The same method, but starting from Example 9, gave, after recrystallisation from ethanol, the title compound as white crystals m.p. : 246-8°C.

| *Analysis* : C₁₆H₁₉N₅O₄S ; 0,5H₂O | | | |
|---|---|---|---|
| *Calculated* : | C, 49.72 ; | H, 5.21 ; | N, 18.12 |
| *Found* : | C, 49.34 ; | H, 5.10 ; | N, 17.97 |

### EXAMPLE 14

### 1-Ethyl-3-methyl-6-(2-ethoxy-5-methanesulfonamidophenyl)-1,5-dihydropyrazolo[3,4-d] pyrimidin-4-one

The same method, but starting from Example 10, gave, after recrystallisation from ethanol, the title compound as white crystals m.p. : 220°C.

| Analysis : C₁₇H₂₁N₅O₄S | | | |
|---|---|---|---|
| Calc : | C, 52.16 ; | H, 5.41 ; | N, 17.89 |
| Found : | C, 52.30 ; | H, 5.34 ; | N, 17.92 |

### EXAMPLE 15

### 1,3-Dimethyl-6-(2-propoxy-5-acetamidophenyl)-1,5-dihydropyrazolo[3,4-d]pyrimidin-4-one

Acetyl chloride (0.3 ml) was added to a solution of Example 7 (0.5 g) in tetrahydrofuran (30 ml) and triethylamine (0.4 ml) and the mixture was stirred 30 minutes at room temperature. The precipitate was filtered off and the solution concentrated in vacuo. The residue was taken up in water and extracted with dichloromethane. The organic layer was washed with water, dried (Na₂SO₄) and concentrated. Recrystallisation from ethanol afforded the title compound as white crystals (400 mg) m.p. : 254-6°C.

| *Analysis* : C₁₈H₂₁N₅O₃ | | | |
|---|---|---|---|
| *Calculated* : | C, 60.82 ; | H, 5.98 ; | N, 19.70 |
| *Found* : | C, 60.87 ; | H, 6.01 ; | N, 19.96 |

The following compounds were obtained in a similar manner :

### EXAMPLE 16

### 1-Ethyl-3-methyl-6-(2-propoxy-5-acetamidophenyl)-1,5-dihydropyrazolo[3,4-d]pyrimidin-4-one

The same method, but starting from acetyl chloride and Example 8, gave, after recrystallisation from ethanol, the title compound as white crystals m.p. : 239-41°C.

| *Analysis* : C₁₉H₂₃N₅O₃ ; 0,5H₂O | | | |
|---|---|---|---|
| *Calculated* : | C, 60.30 ; | H, 6.38 ; | N, 18.50 |
| *Found* : | C, 60.29 ; | H, 6.06 ; | N, 18.75 |

### EXAMPLE 17

### 1-Ethyl-3-methyl-6-(2-ethoxy-5-acetamidophenyl)-1,5-dihydropyrazolo[3,4-d]pyrimidin-4-one

The same method, but starting from Example 10 and acetyl chloride, gave, after recrystallisation from ethanol, the title compound as white crystals m.p. : 250°C.

| *Analysis* : C₁₈H₂₁N₅O₃ | | | |
|---|---|---|---|
| *Calculated* : | C, 60.83 ; | H, 5.96 ; | N, 19.71 |
| *Found* : | C, 60.56 ; | H, 5.92 ; | N, 19.74 |

### EXAMPLE 18

### 1,3-Dimethyl-6-[2-propoxy-5-(4-fluorobenzamido)phenyl]-1,5-dihydropyrazolo[3,4-d]pyrimidin-4-one

The same method, but starting from Example 7 and 4-fluorobenzoyl chloride, gave after recrystallisation from ethanol, the title compound as white crystals m.p. : 259°C.

| *Analysis* : C₂₃H₂₂N₅FO₃ | | | |
|---|---|---|---|
| *Calculated* : | C, 63.44 ; | H, 5.09 ; | N, 16.08 |
| *Found* : | C, 63.68 ; | H, 5.18 ; | N, 15.96 |

### EXAMPLE 19

### 1,3-Dimethyl-6-[2-propoxy-5-((2-thienyl)carboxamido)phenyl]-1,5-dihydropyrazolo[3,4-d]pyrimidin-4-one

The same method, but starting from Example 7 and 2-thienoyl chloride, gave, after recrystallisation from ethanol, the title compound as white crystals m.p. : 265°C.

| *Analysis* : C₂₁H₂₁N₅O₃S | | | |
|---|---|---|---|
| *Calculated* : | C, 59.56 ; | H, 5.06 ; | N, 16.54 |
| *Found* : | C, 59.73 ; | H, 5.01 ; | N, 16.66 |

### EXAMPLE 20

### 1,3-Dimethyl-6-[2-propoxy-5-(bromoacetamido)phenyl]-1,5-dihydropyrazolo[3,4-d]pyrimidin-4-one

The same method, but starting from Example 7 and bromoacetyl chloride, gave, after recrystallisation from ethanol, the title compound as white crystals m.p. : 208-10°C.

| *Analysis* : C₁₈H₂₀BrN₅O₃ | | | |
|---|---|---|---|
| *Calculated* : | C, 49.78 ; | H, 4.64 ; | N, 16.13 |
| *Found* : | C, 50.08 ; | H, 4.67 ; | N, 16.21 |

### EXAMPLE 21

### 1,3-Dimethyl-6-(2-propoxy-5-methoxycarbonylaminophenyl)-1,5-dihydropyrazolo[3,4-d] pyrimidin-4-one

The same method, but starting from Example 7 and methyl chloroformate, gave, after recrystallisation from tetrahydrofuran, the title compound as white crystals m.p. : 252-4°C.

| *Analysis* : C₁₈H₂₁N₅O₄ | | | |
|---|---|---|---|
| *Calculated* : | C, 58.20 ; | H, 5.70 ; | N, 18.86 |
| *Found* : | C, 58.17 ; | H, 5.77 ; | N, 19.28 |

### EXAMPLE 22

### 1,3-Dimethyl-6-(2-propoxy-5-ethoxycarbonylaminophenyl)-1,5-dihydropyrazolo[3,4-d]pyrimidin-4-one

The same method, but starting from Example 7 and ethyl chloroformate (0.3 ml), gave, after recrystallisation from ethanol, the title compound as white crystals m.p. : 220°C.

| *Analysis* : C₁₉H₂₃N₅O₄ | | | |
|---|---|---|---|
| *Calculated* : | C, 59.21 ; | H, 6.01 ; | N, 18.17 |
| *Found* : | C, 59.23 ; | H, 6.12 ; | N, 18.28 |

### EXAMPLE 23

### 1,3-Dimethyl-6-(2-propoxy-5-ureidophenyl)-1,5-dihydropyrazolo[3,4-d]pyrimidin-4-one

Potassium cyanate (1 g) was added to a solution of Example 7 (500 mg) in water (5 ml) and acetic acid (5 ml). After stirring 1 hour at room temperature, the crystals were filtered off, washed with water and dried. Recrystallisation from ethanol provided the title compound as white crystals (300 mg) m.p. : 300°C.

| *Analysis* : C₁₇H₂₀N₆O₃ | | | |
|---|---|---|---|
| *Calculated* : | C, 57.29 ; | H, 5.65 ; | N, 23.58 |
| *Found* : | C, 56.93 ; | H, 5.46 ; | N, 23.34 |

### EXAMPLE 24

### 1,3-Dimethyl-6-(2-propoxy-5-thioureidophenyl)-1,5-dihydro-pyrazolo[3,4-d]pyrimidin-4-one

A mixture of potassium thiocyanate (2 g), Example 7 (500 mg) in water (5 ml) and acetic acid (5 ml) was heated at reflux with stirring during 3 hours. The mixture was then diluted with water and the crystals were filtered off, washed with water, ethanol and dried. Recrystallisation from dimethylformamide afforded the title compound as white crystals (240 mg) m.p : 300°C.

| *Analysis* : C₁₇H₂₀N₅O₂S | | | | |
|---|---|---|---|---|
| *Calculated* : | C, 54.82 ; | H, 5.41 ; | N, 23.06 ; | S, 8.61 |
| *Found* : | C, 54.99 ; | H, 5.54 ; | N, 23.06 ; | S, 9.09 |

### EXAMPLE 25

### 1,3-Dimethyl-6-[2-propoxy-5-(N-cyano-S-methylisothioureido)phenyl]-1,5-dihydro-pyrazolo[3,4-d]pyrimidin-4-one

A solution of Example 7 (1.9 g), cyanoiminodithiocarbonate (3 g) in ethanol (150 ml) was heated under reflux with a slow bubbling of argon during 3 days. The solution was concentrated in vacuo and the residue chromatographed on silica gel. The column was eluted first with toluene/isopropylamine 90/10 and finally the desired product was eluted with dichloromethane/methanol 90/10. After recrystallisation from acetonitrile the title product was obtained as white crystals (0.98 g) m.p : 260°C.

| *Analysis* : C₁₉H₂₁N₇O₂S | | | |
|---|---|---|---|
| *Calculated* : | C, 55.45 ; | H, 5.14 ; | N, 23.85 |
| *Found* : | C, 55.39 ; | H, 5.35 ; | N, 23.41 |

### EXAMPLE 26

### 1,3-Dimethyl-6-[2-propoxy-5-(N-methyl-N-cyanoguanidino)phenyl]-1,5-dihydro-pyrazolo[3,4-d]pyrimidin-4-one

Methylamine gas was bubbled slowly in a solution of Example 25 (300 mg) in ethanol (50 ml) with a gentle reflux during 2 hours. The resulting precipitate was filtered off, washed with ethanol and dried. Recrystallisation from dimethylformamide afforded the title compound as white crystals (250 mg) m.p.: > 300°C.

| *Analysis* : C₁₉H₂₂N₈O₂ | | | |
|---|---|---|---|
| *Calculated* : | C, 57.86 ; | H, 5.62 ; | N, 28.41 |
| *Found* : | C, 57.96 ; | H, 5.69 ; | N, 28.20 |

### EXAMPLE 27

### 1,3-Dimethyl-6-[2-propoxy-5-(N-butyl-N-cyanoguanidino)phenyl]-1,5-dihydro-pyrazolo[3,4-d]pyrimidin-4-one

n-Butylamine (1 ml, excess) was added to a solution of Example 25 (300 mg) in ethanol (50 ml) and the mixture was refluxed during 4 hours and cooled. The resulting precipitate was filtered off and washed with chloroform. Recrystallisation from ethanol afforded the title compound as shiny crystals (115 mg) m.p. : 295°C.

| *Analysis* : C₂₂H₂₈N₈O₂ ; 0,5H₂O | | | |
|---|---|---|---|
| *Calculated* : | C, 59.30 ; | H, 6.56 ; | N, 25.15 |
| *Found* : | C, 59.15 ; | H, 6.39 ; | N, 24.97 |

### EXAMPLE 28

### 1,3-Dimethyl-6-[2-propoxy-5-(4-fluorophenylureido)phenyl]-1,5-dihydro-pyrazolo[3,4-d]pyrimidin-4-one

4-Fluorophenylisocyanate (0.2 g, 1.5 mmol) was added to a solution of Example 7 (0.31 g, 1 mmol) in tetrahydrofuran (40 ml). Crystallisation occured after a few minutes and the mixture was stirred 30 minutes at room temperature. The crystals were filtered off, washed with ether and dried. Recrystallisation from tetrahydrofuran provided the title compound (200 mg) as white crystals. m.p : 280°C.

| *Analysis* : C₂₃H₂₃FN₆O₂ | | | | |
|---|---|---|---|---|
| *Calculated* : | C, 61.32 ; | H, 5.14 ; | N, 18.65 ; | F, 4.21 |
| *Found* : | C, 62.19 ; | H, 5.15 ; | N, 18.61 ; | F, 3.80 |

The following compounds were obtained in a similar manner :

### EXAMPLE 29

### 1,3-Dimethyl-6-(2-propoxy-5-ethoxycarbonylthioureidophenyl)-1,5-dihydro-pyrazolo[3,4-d]pyrimidin-4-one

The same method, but starting from Example 7 and ethoxycarbonylisothiocyanate gave, after recrystallisation from tetrahydrofuran, the title compound m.p : 240-2°C.

| *Analysis* : C₂₀H₂₄N₆O₄S | | | | |
|---|---|---|---|---|
| *Calculated* : | C, 54.03 ; | H, 5.44 ; | N, 18.90 ; | S, 7.21 |
| *Found* : | C, 53.96 ; | H, 5.34 ; | N, 19.28 ; | S, 7.15 |

### EXAMPLE 30

### 1,3-Dimethyl-6-(2-propoxy-5-ethylureidophenyl)-1,5-dihydro-pyrazolo[3,4-d]pyrimidin-4-one

The same method, but starting from Example 7 and ethyl isocyanate, gave, after recrystallisation from ethanol, the title compound as white crystals m.p.: > 300°C.

| *Analysis* : C₁₉H₂₄N₆O₃ | | | |
|---|---|---|---|
| *Calculated* : | C, 59.36 ; | H, 6.29 ; | N, 21.86 |
| *Found* : | C, 59.24 ; | H, 6.33 ; | N, 22.03 |

### EXAMPLE 31

### 1,3-Dimethyl-6-(2-propoxy-5-ethylthioureidophenyl)-1,5-dihydro-pyrazolo[3,4-d]pyrimidin-4-one

The same method, but starting from Example 7 and ethyl isothiocyanate, gave, after recrystallisation from tetrahydrofuran, the title compound as white crystals m.p. : 261°C.

### EXAMPLE 32

### 1,3-Dimethyl-6-(2-propoxy-5-dimethylaminophenyl)-1,5-dihydro-pyrazolo[3,4-d]pyrimidin-4-one

30% formaldehyde solution (3 ml) was added to a solution of Example 7 (0.5 g) in formic acid (3 ml) and the mixture was heated overnight at 80°C. The solution was then poured into water, neutralised with sodium hydroxide and the mixture extracted with dichloromethane. The organic layer was washed with water, dried (Na₂SO₄) and concentrated to give a yellow oil that was purified by chromatography on silica gel with the mixture dichloromethane/methanol 95/5 as eluent. Recrystallisation from ethanol afforded the title compound as yellow crystals (125 mg) m.p. : 180°C.

| *Analysis* : C₁₈H₂₃N₅O₂ | | | |
|---|---|---|---|
| *Calculated* : | C, 63.32 ; | H, 6.76 ; | N, 20.52 |
| *Found* : | C, 62.75 ; | H, 6.77 ; | N, 20.42 |

The following compound is obtained in a similar manner :

### EXAMPLE 33

### 1-Ethyl-3-methyl-6-(2-propoxy-5-dimethylaminophenyl)-1,5-dihydro-pyrazolo[3,4-d]pyrimidin-4-one

The same method, but starting from Example 8, gave, after recrystallisation from acetonitrile, the title compound as yellow crystals m.p. : 153°C.

| *Analysis* : C₁₉H₂₅N₅O₂ | | | |
|---|---|---|---|
| *Calculated* : | C, 64.20 ; | H, 7.09 ; | N, 19.70 |
| *Found* : | C, 64.11 ; | H, 7.14 ; | N, 19.77 |

### EXAMPLE 34

### 1,3-Dimethyl-6-(5-cyano-2-propoxyphenyl)-1,5-dihydro-pyrazolo[3,4-d]pyrimidin-4-one

A mixture of 1,3-dimethyl-6-(5-bromo-2-propoxyphenyl) -1,5-dihydro-pyrazolo[3,4-d]pyrimidin-4-one (prepared according to the method described in WO93/07149; 0.34 g, 0.9 mmol), sodium cyanide (0.045 g, 0.9 mmol), 18-crown-6 (0.24g, 0.9 mmol) and tetrakis(triphenylphosphine)palladium (0) (0.52 g, 0.45 mmol) in tetrahydrofuran (20 ml) and ethanol (20 ml) was heated under reflux for 4 hours and then concentrated under reduced pressure. The residue was diluted with water, extracted with dichloromethane. The organic phase was dried (Na₂SO₄) and concentrated in vacuo to give a solid that was chromatographied on silica gel with a mixture of dichloromethane/methanol (98/2) as eluent. After crystallisation from isopropanol, the title compound was obtained as white crystals (0.14 g) m.p. : 208°C.

The following compound is obtained in a similar manner :

### EXAMPLE 35

### 1-Ethyl-3-methyl-6-(5-cyano-2-propoxyphenyl)-1,5-dihydro-pyrazolo[3,4-d]pyrimidin-4-one

The same method, but starting from 1-ethyl-3-methyl-6-(5-bromo-2-propoxyphenyl)-1,5-dihydro-pyrazolo[3,4-d]pyrimidin-4-one, (prepared according to the method described in WO93/07149) gave, after crystallisation from isopropanol, the title compound as white crystals m.p. : 190°C.

| *Analysis* : C₁₈H₁₉N₅O₂ | | | |
|---|---|---|---|
| *Calculated* : | C, 64.08 ; | H, 5.68 ; | N, 20.76 |
| *Found* : | C, 64.22 ; | H, 5.79 ; | N, 20.64 |

### EXAMPLE 36

### 1-Ethyl-3-methyl-6-(2-propoxy-5-thiocarbamoylphenyl)-1,5-dihydro-pyrazolo[3,4-d]pyrimidin-4-one

A mixture of Example 35 (0.85 g, 2.52 mmol), diethyldithiophosphate (0.5 ml, 3 mmol) and a drop of water was stirred at room temperature during 24 hours and then poured into water. The precipitate was filtered off, washed with water and dried. After crystallisation from methanol, the title compound was obtained as yellow crystals (0.58 g) m.p. : 256°C.

| *Analysis* : C₁₈H₂₁N₅O₂S | | | |
|---|---|---|---|
| *Calculated* : | C, 58.20 ; | H, 5.70 ; | N, 18.85 |
| *Found* : | C, 58.37 ; | H, 5.78 ; | N, 18.73 |

### EXAMPLE 37

### 1-Ethyl-3-methyl-6-[2-propoxy-5-(4-methyl-2-thiazolyl)phenyl]-1,5-dihydro-pyrazolo[3,4-d] pyrimidin-4-one

A mixture of Example 36 (0.45 g, 1.2 mmol) and chloroacetone (0.195 ml, 2.4 mmol) in absolute ethanol (60 ml) was heated under reflux during 16 hours. After concentration under reduced pressure, the residue was treated with water and extracted with dichloromethane, the organic layer was washed with water, dried (Na₂SO₄) and concentrated. The residue was purified by column chromatography on silica gel eluting with dichloromethane/methanol (95/5). After crystallisation from isopropanol, the title compound was obtained as off white crystals (0.06 g) m.p. : 215°C.

| *Analysis* : C₂₁H₂₃N₅O₂S₁ | | | |
|---|---|---|---|
| *Calculated* : | C, 61.59 ; | H, 5.66 ; | N, 17.10 |
| *Found* : | C, 61.20 ; | H, 5.67 ; | N, 16.89 |

### EXAMPLE 38

### 1-Ethyl-3-methyl-6-(2-propoxy-5-hydroxyamidinophenyl)-1,5-dihydro-pyrazolo[3,4-d]pyrimidin-4-one

A mixture of Example 35 (1 g, 3 mmol), hydroxylamine hydrochloride (0.82 g, 12 mmol), potassium carbonate (1.025 g, 7.5 mmol) in methanol (50 ml) and water (50 ml) was heated under reflux during 4 hours, and poured into water. The precipitate was filtered off, washed with water and dried. After crystallisation from methanol/dimethylformamide, the title compound was obtained as white crystals (0.47 g) m.p.: > 260°C.

| *Analysis* : C₁₈H₂₂N₆O₃ | | | |
|---|---|---|---|
| *Calculated* : | C, 58.37 ; | H, 5.99 ; | N, 22.69 |
| *Found* : | C, 58.29 ; | H, 5.95 ; | N, 22.62 |

### EXAMPLE 39

### 1,3-Dimethyl-6-[2-propoxy-5-(1-N-methyl-3-hydantoinylmethyl)phenyl]-1,5-dihydro-pyrazolo[3,4-d] pyrimidin-4-one

To a suspension of 60% sodium hydride in mineral oil (54 mg, 1.365 mmol) in dimethylformamide (5 ml) was added dropwise a solution of 1-N-methylhydantoin (146 mg, 1.3 mmol) and the mixture was stirred at room temperature during 15 minutes. A solution of Intermediate 16 (0.5 g, 1.3 mmol) in dimethylformamide (10 ml) was added dropwise and the mixture was stirred at 50°C during 1.5 hours, and then concentrated under reduced pressure. The residue was heated with water, extracted with dichloromethane. The organic layer was dried (Na₂SO₄) and concentrated to leave a solid which was purified by column chromatography on silica gel eluting with dichloromethane/methanol (95/5). After crystallisation from methanol, the title compound was obtained as white crystals (0.09 g) m.p. : 236°C.

| *Analysis* : C₂₁H₂₄N₆O₄ | | | |
|---|---|---|---|
| *Calculated* : | C, 59.42 ; | H, 5.70 ; | N, 19.80 |
| *Found* : | C, 59.38 ; | H, 5.74 ; | N, 19.81 |

### EXAMPLE 40

### 1,3-Dimethyl-6-[2-propoxy-5-(2-phenyl-4-thiazolyl)phenyl]-1,5-dihydro-pyrazolo[3,4-d]pyrimidin-4-one

A mixture of 1,3-dimethyl-6-(2-propoxy-5-bromoacetylphenyl)-1,5-dihydro-pyrazolo[3,4-d]pyrimidin-4-one (prepared according to the method described in WO93/07149; 0.4 g, 0.95 mmol) and thiobenzamide (0.13 g, 0.95 mmol) in absolute ethanol (50 ml) was heated under reflux during 1 hour and concentrated under reduced pressure. The residue was heated with water, extracted with dichloromethane. The organic phase was dried (Na₂SO₄) and concentrated to leave a solid which was purified by column chromatography on silica gel eluting with dichloromethane/methanol (95/5). After crystallisation from methanol, the title compound was obtained as white crystals (0.12 g) m.p. : 200°C.

| *Analysis* : C₂₅H₂₃N₅O₂S₁ | | | |
|---|---|---|---|
| *Calculated* : | C, 65.63 ; | H, 5.07 ; | N, 15.31 |
| *Found* : | C, 65.89 ; | H, 5.09 ; | N, 15.45 |

### EXAMPLE 41

### 1,3-Dimethyl-6-(2-propoxy-5-trifluoroacetamidophenyl)-1,5-dihydro-pyrazolo[3,4-d]pyrimidin-4-one

Trifluoroacetic anhydride (0.5 ml, 4 ml) was added to a solution of Example 7 (310 mg, 1 mmol) and triethylamine (0.5 ml) in tetrahydrofuran (30 ml) and the mixture was stirred one hour at room temperature. The solution was then concentrated in vacuo and the residue taken up in water and extracted with dichloromethane. The organic layer was washed with water, dried (Na₂SO₄) and concentrated. Recrystallisation from ethanol yielded the title compound as white crystals (310 mg) m.p. : 215°C.

| *Analysis* : C₁₈H₁₈F₃N₅O₃ | | | |
|---|---|---|---|
| *Calculated* : | C, 52.81 ; | H, 4.43 ; | N, 17.11 |
| *Found* : | C, 52.72 ; | H, 4.32 ; | N, 16.99 |

### EXAMPLE 42

### 1,3-Dimethyl-6-(2-isopropoxy-5-nitrophenyl)-1,5-dihydro-pyrazolo[3,4-d]pyrimidin-4-one

Intermediate 6 (0.4 g ; 1.3 mmol) was added portionwise to a solution of nitric acid (15 ml ; d = 1.42) and concentrated sulfuric acid (15 ml) kept at 0°C. The mixture was stirred at 0°C during 15 minutes and then poured into ice. The precipitate was filtered off and washed with water. After crystallization from methanol/dimethylformamide, the title compound was obtained as yellow crystals (0.18 g) m.p.: 240°C.

| *Analysis* : C₁₆H₁₇N₅O₄ | | | |
|---|---|---|---|
| *Calculated* : | C, 55.97 ; | H, 4.99 ; | N, 20.40 |
| *Found* : | C, 55.60 ; | H, 5.04 ; | N, 20.12 |

The following compound was obtained in a similar manner :

### EXAMPLE 43

### 1,3-Dimethyl-6-(4-methyl-5-nitro-2-propoxyphenyl)-1,5-dihydro-pyrazolo[3,4-d] pyrimidin-4-one

The same method, but starting from Intermediate 7 gave, after crystallization from methanol/dimethylformamide, the title compound as yellow crystals m.p. : 212°C.

| *Analysis* : C₁₇H₁₉N₅O₄ | | | |
|---|---|---|---|
| *Calculated* : | C, 57.14 ; | H, 5.36 ; | N, 19.60 |
| *Found* : | C, 55.85 ; | H, 5.36 ; | N, 19.29 |

### EXAMPLE 44

### 1,3-Dimethyl-6-(5-amino-4-methyl-2-propoxyphenyl)-1,5-dihydro-pyrazolo[3,4-d] pyrimidin-4-one

The same method as used in the preparation of Example 7, but starting from Example 43, gave, after crystallization from water/dimethylformamide, the title compound as light brown crystals m.p. : 218°C.

### EXAMPLE 45

### 1,3-Dimethyl-6-[2-propoxy-5-(4-methylpiperazin-1-yl)acetylamino]phenyl-1,5-dihydro-pyrazolo[3,4-d]pyrimidin-4-one

A mixture of Example 20 (300 mg; 0.69 mM) and N-methylpiperazine (1 g) was heated at 90°C during five minutes without solvent. The melted mixture was diluted with water (20 ml) and extracted with dichloromethane. After washing with water, drying (Na₂SO₄) and evaporation, and crystallization from ethyl acetate, the title compound was obtained as white crystals (150 mg) m.p.: 163°C.

| *Analysis* : C₂₃H₃₁N₇O₃, 1H₂O | | | |
|---|---|---|---|
| *Calculated* : | C, 58.58 ; | H, 7.05 ; | N, 20.79 |
| *Found* : | C, 58.16 ; | H, 6.91 ; | N, 20.22 |

### EXAMPLE 46

### 1,3-Dimethyl-6-[2-propoxy-5-(2-imidazolidinethione-1-ylmethyl)phenyl]-1,5-dihydro-pyrazolo[3,4-d]pyrimidin-4-one

The same method as used in the preparation of Example 39, but starting from 2-imidazolidinethione, gave, after crystallization from isopropanol the title compound as white crystals m.p. = 186-188°C.

### EXAMPLE 47

### 1,3-Dimethyl-6-[2-propoxy-5-(N-succinimidylmethyl)phenyl]-1,5-dihydro-pyrazolo[3,4-d]pyrimidin-4-one

The same method as used in the preparation of Example 39, but starting from succinimide, gave, after crystallization from methanol, the title compound as white crystals m.p. : 250°C.

### EXAMPLE 48

### 1-Ethyl-3-methyl-6-(2-propoxy-5-hydrazidophenyl)-1,5-dihydro-pyrazolo[3,4-d] pyrimidin-4-one

A mixture of 1-ethyl-3-methyl-6-(2-propoxy-5-methylcarboxyphenyl)-1,5-dihydro-pyrazolo[3,4-d]pyrimidin-4-one (prepared according to the method described in WO93/07149; 0.9 g ; 2.43 mmol) and hydrazine hydrate (1.4 ml) in ethanol was heated under reflux during 16 hours and concentrated under reduced pressure. The residue was treated with water and extracted with dichloromethane ; the organic layer was dried (Na₂SO₄) and concentrated. The residue was crystallized from methanol to afford the title compound as white crystals (0.76 g) m.p.: 264°C.

| *Analysis* : C₁₈H₂₂N₆O₃ | | | |
|---|---|---|---|
| *Calculated* : | C, 58.37 ; | H, 5.99 ; | N, 22.69 |
| *Found* : | C, 58.47 ; | H, 5.90 ; | N, 22.65 |

### EXAMPLE 49

### 1-Ethyl-3-methyl-6-[2-propoxy-5-(3-phenyl-1,2,4-triazol-5-yl)phenyl]-1,5-dihydro-pyrazolo[3,4-d]pyrimidin-4-one

A mixture of Example 48 (0.5 g ; 1.35 mmol) and thiobenzamide (0.185 g ; 1.35 mmol) in xylene (15 ml) was heated under reflux during 24 hours and concentrated under reduced pressure. The residue was treated with water, extracted with dichloromethane, the organic layer was dried (Na₂SO₄) and concentrated under reduced pressure to leave an oil which was purified by column chromatography on silica gel eluting with dichloromethane/methanol (98/2). After crystallization from isopropanol, the title compound was obtained as white crystals (0.04 g).

### EXAMPLE 50

### 1-Ethyl-3-methyl-6-[2-propoxy-5-(acetylaminoacetylamino)phenyl]-1,5-dihydro-pyrazolo[3,4-d]pyrimidin-4-one

A mixture of Example 8 (0.8 g ; 2.44 mmol), N-acetylglycine (0.62 g ; 5.3 mmol) and diphenylphosphorylazide (2.4 ml ; 11 mmol) in dichloromethane (100 ml) was stirred at room temperature for 48 hours, and then poured into water. After extraction with dichloromethane, the organic layer was dried (Na₂SO₄) and concentrated under reduced pressure to leave an oil which was purified by column chromatography on silica gel eluting with dichloromethane/methanol (95/5). After crystallization from isopropanol, the title compound was obtained as light pink crystals (0.23 g) m.p.: > 260°C.

| *Analysis* : C₂₁H₂₆N₆O₄ | | | |
|---|---|---|---|
| *Calculated* : | C, 59.14 ; | H, 6.15 ; | N, 19.71 |
| *Found* : | C, 58.70 ; | H, 6.14 ; | N, 19.63 |

### EXAMPLE 51

### 1,3-Dimethyl-6-[2-propoxy-5-(2-methyl-4-thiazolyl)phenyl]-1,5-dihydro-pyrazolo[3,4-d]pyrimidin-4-one

The same method as used in the preparation of Example 40, but starting from thioacetamide, gave, after crystallization from isopropanol, the title compound as white crystals m.p.: 204°C.

| *Analysis* : C₂₀H₂₁N₅O₂S | | | |
|---|---|---|---|
| *Calculated* : | C, 60.74 ; | H, 5.35 ; | N, 17.71 |
| *Found* : | C, 59.79; | H, 5.31; | N, 17.58 |

### EXAMPLE 52

### 1-(2,2,2-Trifluoroethyl)-3-methyl-6-(2-propoxyphenyl)-1,5-dihydro-pyrazolo [3,4-d]pyrimidin-4-one

2-Propoxybenzoyl chloride (2.10g) was added to a solution of Intermediate 8 (2.04g) in pyridine (50 ml). The mixture was heated 2 hours at 70°C, concentrated, heated with water and extracted with dicholoromethane to give a gummy solid that crystallised by treatment with diisopropyl ether. This product was heated with stirring with 1N sodium hydroxide (20 ml), water (20 ml) and 30% hydrogen peroxide (2.5 ml) during 7 hours. The solution was diluted with water, filtered and made acidic with dilute hydrochloric acid. The resulting precipitate was filtered off, washed with water, dried and recrystallised with ethanol to give the title compound as white crystals (0.34 g) m.p. : 182°C.

| *Analysis* : C₁₇H₁₇F₃N₃O₂ | | | | |
|---|---|---|---|---|
| *Calculated* : | C, 55.73 ; | H, 4.68 ; | N, 15.29 ; | F, 15.55 |
| *Found* : | C, 55.83 ; | H, 4.61 ; | N, 15.44 ; | F, 13.73 |

### EXAMPLE 53

### 1-Benzyl-3-methyl-6-(2-propoxyphenyl)-1,5-dihydropyrazolo[3,4-d]pyrimidin-4-one

The same method as used in the preparation of Example 2, but starting from 2-propoxybenzoyl chloride and Intermediate 10 gave, after crystallisation from methanol, the title compound as yellow crystals mp : 147°C.

| *Analysis* : C₂₂H₂₂N₄O₂ | | | |
|---|---|---|---|
| *Calculated* : | C, 70.57 ; | H, 5.92 ; | N, 14.96 |
| *Found* : | C, 69.07 ; | H, 5.93 ; | N, 14.86 |

### EXAMPLE 54

### 1-Benzyl-3-methyl-6-(2-propoxy-5-nitrophenyl)-1,5-dihydropyrazolo[3,4-d]pyrimidin-4-one

The same method as used in the preparation of Example 2, but starting from 2-propoxy-5-nitro-benzoyl chloride and Intermediate 10 gave, after crystallisation from diisopropylether, the title compound as white crystals mp: 136°C.

| *Analysis* : C₂₂H₂₁N₅O₄ | | | |
|---|---|---|---|
| *Calculated* : | C, 63.00 ; | H, 5.05 ; | N, 16.70 |
| *Found* : | C, 63.26 ; | H, 5.26 ; | N, 16.04 |

### EXAMPLE 55

### 1-(3-Pyridylmethyl)-3-methyl-6-(2-propoxyphenyl)-1,5-dihydropyrazolo[3,4-d]pyrimidin-4-one

The same method as used in the preparation of Example 2, but starting from 2-propoxybenzoyl chloride and Intermediate 11 gave, after crystallisation from isopropanol, the title compound as white crystals mp: 160-162°C.

| *Analysis* : C₂₁H₂₁N₅O₂ | | | |
|---|---|---|---|
| *Calculated* : | C, 67.18 ; | H, 5.64 ; | N, 18.65 |
| *Found* : | C, 67.21 ; | H, 5.66 ; | N, 18.56 |

### EXAMPLE 56

### 1-(4-Pyridylmethyl)-3-methyl-6-(2-propoxyphenyl)-1,5-dihydropyrazolo[3,4-d]pyrimidin-4-one

The same method as used in the preparation of Example 2, but starting from 2-propoxybenzoyl chloride and Intermediate 12 gave, after crystallisation from ethyl acetate/diisopropylether, the title compound as crystals mp : ca. 120°C.
NMR (DMSO, 250 MHz, ppm) 1.0 (t,3H, CH₃(OPr)) ; 1.8 (m, 2H, CH₂ (OPr)) ; 2.5 (s, 3H, CH₃) ; 4.2 (t, 2H, OCH₂) ; 5.8 (s, 2H, CH₂py) ; 7.1 to 8.9 (8HAr); 12.0 (s, 1H, NH)

### EXAMPLE 57

### 1-(2-Pyridylmethyl)-3-methyl-6-(2-propoxyphenyl)-1,5-dihydropyrazolo[3,4-d]pyrimidin-4-one

The same method as used in the preparation of Example 2, but starting from 2-propoxybenzoyl chloride and Intermediate 13 gave, after crystallisation from isopropanol, the title compound as white crystals mp: 158°C.

| *Analysis* : C₂₁H₂₁N₅O₂ | | | |
|---|---|---|---|
| *Calculated* : | C, 67.18 ; | H, 5.64 ; | N, 18.65 |
| *Found* : | C, 67.16 ; | H, 5.60 ; | N, 18.84 |

### EXAMPLE 58

### 1-(3,4-Methylenedioxybenzyl)-3-methyl-6-(2-propoxyphenyl)-1,5-dihydropyrazolo[3,4-d]pyrimidin-4-one

The same method as used in the preparation of Example 2, but starting from 2-propoxybenzoyl chloride and Intermediate 14 gave, after crystallisation from isopropanol, the title compound as white crystals mp: 146°C.

| *Analysis* : C₂₃H₂₂N₄O₄ | | | |
|---|---|---|---|
| *Calculated* : | C, 66.02 ; | H, 5.30 ; | N, 13.39 |
| *Found* : | C, 65.42 ; | H, 5.28 ; | N, 13.25 |

### EXAMPLE 59

### 1,3-Dimethyl-6-(2-propoxy-5-thiocarbamoylphenyl)-1,5-dihydropyrazolo[3,4-d]pyrimidin-4-one

The same method as used in the preparation of Example 36, but starting from Example 34, gave, after crystallisation from methanol, the title compound as yellow crystals mp : > 260°C.

### EXAMPLE 60

### 1,3-Dimethyl-6-[2-propoxy-5-(4-methyl-2-thiazolyl)phenyl]-1,5-dihydropyrazolo[3,4-d]pyrimidin-4-one

The same method as used in the preparation of Example 37, but starting from Example 59 gave, after crystallisation from ethanol, the title compound as white crystals mp : 230°C.

| *Analysis* : C₂₀H₂₁N₅O₂S₁ | | | |
|---|---|---|---|
| *Calculated* : | C, 60.74 ; | H, 5.35 ; | N, 17.71 |
| *Found* : | C, 60.69 ; | H, 5.35 ; | N, 17.67 |

### EXAMPLE 61

### 1,3-Dimethyl-6-[2-propoxy-5-(4-phenyl-2-thiazolyl)phenyl]-1,5-dihydropyrazolo[3,4-d]pyrimidin-4-one

The same method as used in the preparation of Example 37, but starting from Example 59 and chloroacetophenone, gave, after crystallisation from ethanol, the title compound as crystals mp : 166°C.

| *Analysis* : C₂₅H₂₃N₅O₂S₁ | | | |
|---|---|---|---|
| *Calculated* : | C, 65.63 ; | H, 5.07 ; | N, 15.31 |
| *Found* : | C, 64.53 ; | H,5.07 ; | N, 14.97 |

### EXAMPLE 62

### 1,3-Dimethyl-6-[2-propoxy-5-(2-thiazolyl)phenyl]-1,5-dihydropyrazolo[3,4-d]pyrimidin-4-one

A mixture of Example 59 (0.35g), bromoacetaldeyde (0.24g) and potassium carbonate (0.4g) in DMF (20 ml) was stirred at room temperature overnight. After concentration under reduced pressure, the residue was treated with water and extracted with dichloromethane, the organic layer was washed with water, dried (Na₂SO₄) and concentrated. The residue was treated with trifluoroacetic anhydride (0.5 ml) in dichloromethane with stirring during 1 hour at room temperature. Water was then added. After extraction with dichloromethane, the organic layer was dried (Na₂SO₄) and concentrated. The residue was purified by column chromatography on silica gel eluting with dichloromethane/methanol (97/3). After crystallisation from water/DMF, the title compound was obtained as crystals (0.018g) mp : 209°C.

| *Analysis* : C₁₉H₁₉N₅O₂S, 0.5 H₂O | | | |
|---|---|---|---|
| *Calculated* : | C, 58.46 ; | H, 5.13 ; | N, 17.95 |
| *Found* : | C, 58.42 ; | H, 5.10 ; | N, 17.78 |

### EXAMPLE 63

### 1-Ethyl-3-methyl-6-[2-propoxy-5-(2-methyl-4-thiazolyl)phenyl]-1,5-dihydropyrazolo[3,4-d]pyrimidin-4-one

The same method as used in the preparation of Example 40, but starting from 1-ethyl-3-methyl-6-(2-propoxy-5-bromoacetylphenyl)-1,5-dihydropyrazolo[3,4-d]pyrimidin-4-one (prepared according to the method described in W093/07149) and thioacetamide, gave, after crystallisation from isopropanol, the title compound as white crystals mp : 183°C.

| *Analysis* : C₂₁H₂₃N₅O₂S₁ | | | |
|---|---|---|---|
| *Calculated* : | C, 61.59 ; | H, 5.66 ; | N, 17.10 |
| *Found* : | C, 61.44 ; | H, 5.60 ; | N, 17.39 |

### EXAMPLE 64

### 1-Ethyl-3-methyl-6-[2-propoxy-5-(2-phenyl-4-thiazolyl)phenyl]-1,5-dihydropyrazolo[3,4-d] pyrimidin-4-one

The same method as used in the preparation of Example 40, but starting from 1-ethyl-3-methyl-6-(2-propoxy-5-bromoacetylphenyl)-1,5-dihydropyrazolo[3,4- d]pyrimidin-4-one (prepared according to the method described in W093/07149), gave, after crystallisation from isopropanol, the title compound as white crystals mp : 217°C.

| *Analysis* : C₂₆H₂₅N₅O₂S | | | |
|---|---|---|---|
| *Calculated* : | C, 66.22 ; | H, 5.34 ; | N, 14.85 |
| *Found* : | C, 66.00 ; | H, 5.32 ; | N, 15.09 |

### EXAMPLE 65

### 1-Ethyl-3-methyl-6-[2-propoxy-5-(2-(3-pyridyl)-4-thiazolyl)phenyl]-1,5-dihydropyrazolo[3,4-d]pyrimidin-4-one

The same method as used in the preparation of Example 40, but starting from 1-ethyl-3-methyl-6-(2-propoxy-5-bromoacetylphenyl)-1,5-dihydropyrazolo[3,4-d]pyrimidin-4-one (prepared according to the method described in W093/071 49) and thionicotinamide, gave, after crystallisation from isopropanol, the title compound as crystals mp : 219°C.

| *Analysis* : C₂₅H₂₄N₆O₂S₁ | | | |
|---|---|---|---|
| *Calculated* : | C, 63.54 ; | H, 5.12 ; | N, 17.78 |
| *Found* : | C, 62.94 ; | H, 5.13 ; | N, 17.57 |

### EXAMPLE 66

### 1,3-Dimethyl-6-(2-propoxy-5-hydrazidophenyl)-1,5-dihydropyrazolo[3,4-d]pyrimidin-4-one

The same method as used in the preparation of Example 48 but starting from 1,3-dimethyl-6-(2-propoxy-5-methylcarboxyphenyl)-1,5-dihydropyrazolo[3,4-d]pyrimidin-4-one (prepared according to the method described in W093/07149) gave the title compound as a white solid.
NMR (DMSO, 250 MHz, ppm) : 0.95 (t, 3H, CH₃ (OPr)) ; 1.75 (m, 2H, CH₂ (OPr)) ; 2.4 (s, 3H, CH₃) ; 3.85 (s, 3H, N-CH₃) ; 4.1 (t, 2H, O-CH₂) ; 7.2 (d, 1H, H₃Ar) ; 7.95 (dd, 1H, H₄Ar) ; 8.2 (sd, 1H, H₆Ar) ; 9.8 (s, 1H, NH).

### EXAMPLE 67

### 1,3-Dimethyl-6-[2-propoxy-5-(3-phenyl-1,2,4-triazol-5-yl)phenyl]-1,5-dihydropyrazolo[3,4-d] pyrimidin-4-one

The same method as used in the preparation of Example 49, but starting from Example 66 gave, after crystallisation from isopropanol/diisopropylether, the title compound as white crystals mp : 250°C.

| *Analysis* : C₂₄H₂₃N₇O₂ | | | |
|---|---|---|---|
| *Calculated* : | C, 65.29 ; | H, 5.25 ; | N, 22.21 |
| *Found* : | C, 65.45 ; | H, 5.24 ; | N, 19.04 |

### EXAMPLE 68

### 1,3-Dimethyl-6-[2-propoxy-5-(2-thienyl)phenyl]-1,5-dihydropyrazolo[3,4-d]pyrimidin-4-one

A mixture Intermediate 5, 2-(trimethyl-tin)thiophene (2.5g) and bis(triphenylphosphine)palladium (II) chloride (360 mg) in tetrahydrofuran (50 ml) was heated under reflux during 20 hours and then concentrated under reduced pressure. The residue was diluted with water, extracted with dichloromethane. The organic phase was dried (Na₂SO₄) and concentrated in vacuo. The residue was purified by column chromatography on silica gel, eluting with dichloromethane/methanol (99/1). After crystallisation from diisopropylether/isopropanol, the title compound was obtained as crystals (0.17 g) mp : 184°C.

| *Analysis* : C₂₀H₂₀N₄O₂S₁ | | | |
|---|---|---|---|
| *Calculated* : | C, 63.14 ; | H, 5.30 ; | N, 14.73 |
| *Found* : | C, 63.33 ; | H, 5.19 ; | N, 14.57 |

### EXAMPLE 69

### 1,3-Dimethyl-6-[2-propoxy-5-(5-methyl-2-thienyl)phenyl]-1,5-dihydropyrazolo[3,4-d]pyrimidin-4-one

The same method as used in the preparation of Example 68, but starting from 5-methyl-2-(trimethyltin)thiophene, gave after crystallisation from diisopropylether/isopropanol, the title compound as white crystals mp : 178°C.

| *Analysis* : C₂₁H₂₂N₄O₂S₁ | | | |
|---|---|---|---|
| *Calculated* : | C, 63.94 ; | H, 5.62 ; | N, 14.20 |
| *Found* : | C, 63.02 ; | H, 5.56 ; | N, 14.11 |

### BIOLOGICAL ACTIVITY

Compounds of the invention were assayed according to the procedure described hereinabove. Results show that compounds of the invention are potent inhibitors of cGMP specific PDE as demonstrated by the IC₅₀ values for a representative selection of compounds of the invention hereinafter. Thus, compounds of Examples 7, 11, 15, 18, 19, 22, 30, 31, 37, 41, 51, 63, 65, 67 and 69 above have IC₅₀ values in the range of 1 to 10nM. In the same assay zaprinast¹ has an IC₅₀ of 200nM.
1. Zaprinast is a known selective inhibitor of cGMP specific PDE (cf. T Saeki and I Saito in Biochemical Pharmacology, 46(5), p833-839, 1993).

## Claims

1. Compounds of formula (I) 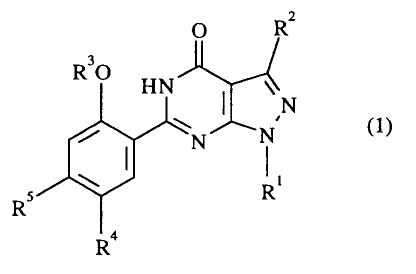 and salts and solvates (e.g. hydrates) thereof, in which:
R¹ represents arylmethyl or C₁₋₆alkyl optionally substituted by one or more fluorine atoms;
R² represents methyl;
R³ represents C₂₋₄alkyl;
R⁴ represents nitro, cyano, C₁₋₆alkoxy, C(=X)NR⁶R⁷, NR⁸R⁹, (CH₂)ₘNR¹⁰C(=Y)R¹¹ or a 5-membered heterocyclic ring selected from thienyl, thiazolyl and 1,2,4-triazolyl each ring optionally substituted by a C₁₋₄alkyl or aryl group; or when R¹ is arylmethyl or C₁₋₆alkyl substituted by one or more fluorine atoms then R⁴ may also represent hydrogen;
R⁵ represents hydrogen or C₁₋₆alkyl;
R⁶ represents hydrogen or C₁₋₆alkyl;
R⁷ represents hydrogen, amino, hydroxyl, C₁₋₆alkyl, aryl or arylC₁₋ ₄alkyl;
R⁸ represents hydrogen or C₁₋₆alkyl;
R⁹ represents hydrogen, C₁₋₆alkyl, SO₂R¹², CO₂R¹², C(=NCN)SR¹² or C(=NCN)NR¹³R¹⁴;
R¹⁰ represents hydrogen or C₁₋₆alkyl;
R¹¹ represents C₁₋₆alkyl optionally substituted by one or more halogen atoms, or R¹¹ represents aryl, arylC₁₋₄alkyl, thienyl, NR¹⁵R¹⁶, CH₂NR¹⁷R¹⁸ or R¹⁰ and R¹¹ together represent -A(CH₂)ₙ-;
R¹² represents C₁₋₆alkyl, aryl or arylC₁₋₄alkyl;
R¹³ represents hydrogen or C₁₋₆alkyl;
R¹⁴ represents hydrogen, C₁₋₆alkyl, aryl, arylC₁₋₄alkyl or R¹³ and R¹⁴ together with the nitrogen atom to which they are attached form a morpholine, piperazine or N-C₁₋₄alkylpiperazine ring;
R¹⁵ represents hydrogen or C₁₋₆alkyl or R¹⁰ and R¹⁵ together represent -A(CH₂)ₙ-;
R¹⁶ represents hydrogen, C₁₋₆alkyl, aryl, arylC₁₋₄alkyl, CO₂R¹², CH₂CO₂R¹² or R¹⁵ and R¹⁶ together with the nitrogen atom to which they are attached form a morpholine, piperazine or N-C₁₋ ₄alkylpiperazine ring;
R¹⁷ represents hydrogen or C₁₋₆alkyl;
R¹⁸ represents hydrogen, C₁₋₆alkyl, aryl, arylC₁₋₄alkyl, COR¹² or R¹⁷ and R¹⁸ together with the nitrogen atom to which they are attached form a morpholine, piperazine or N-C₁₋₄alkylpiperazine ring;
A represents CH₂ or C=O;
m represents zero or 1;
n represents 1,2 or 3;
X represents S or NH, or when R⁷ represents amino then X may also represent O;
Y represents O or S; for use in therapy.

2. Compounds of formula (I) 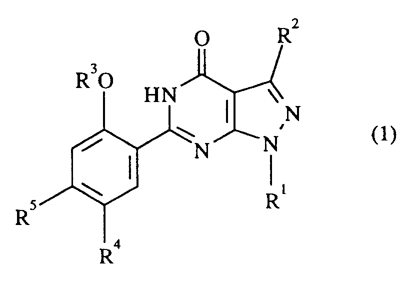 and salts and solvates (e.g. hydrates) thereof, in which:
R¹ represents arylmethyl or C₁₋₆alkyl optionally substituted by one or more fluorine atoms;
R² represents methyl;
R³ represents C₂₋₄alkyl;
R⁴ represents nitro, cyano, C₁₋₆alkoxy, C(=X)NR⁶R⁷, NR⁸R⁹, (CH₂)ₘNR¹⁰C(=Y)R¹¹ or a 5-membered heterocyclic ring selected from thienyl, thiazolyl and 1,2,4-triazolyl each ring optionally substituted by a C₁₋₄alkyl or aryl group; or when R¹ is arylmethyl or C₁₋₆alkyl substituted by one or more fluorine atoms then R⁴ may also represent hydrogen;
R⁵ represents hydrogen or C₁₋₆alkyl;
R⁶ represents hydrogen or C₁₋₆alkyl;
R⁷ represents hydrogen, amino, hydroxyl, C₁₋₆alkyl, aryl or arylC₁₋ ₄alkyl;
R⁸ represents hydrogen or C₁₋₆alkyl;
R⁹ represents hydrogen, C₁₋₆alkyl, SO₂R¹², CO₂R¹², C(=NCN)SR¹² or C(=NCN)NR¹³R¹⁴;
R¹⁰ represents hydrogen or C₁₋₆alkyl;
R¹¹ represents C₁₋₆alkyl optionally substituted by one or more halogen atoms, or R¹¹ represents aryl, arylC₁₋₄alkyl, thienyl, NR¹⁵R¹⁶, CH₂NR¹⁷R¹⁸ or R¹⁰ and R¹¹ together represent -A(CH₂)ₙ-;
R¹² represents C₁₋₆alkyl, aryl or arylC₁₋₄alkyl;
R¹³ represents hydrogen or C₁₋₆alkyl;
R¹⁴ represents hydrogen, C₁₋₆alkyl, aryl, arylC₁₋₄alkyl or R¹³ and R¹⁴ together with the nitrogen atom to which they are attached form a morpholine, piperazine or N-C₁₋₄alkylpiperazine ring;
R¹⁵ represents hydrogen or C₁₋₆alkyl or R¹⁰ and R¹⁵ together represent -A(CH₂)ₙ-;
R¹⁶ represents hydrogen, C₁₋₆alkyl, aryl, arylC₁₋₄alkyl, CO₂R¹², CH₂CO₂R¹² or R¹⁵ and R¹⁶ together with the nitrogen atom to which they are attached form a morpholine, piperazine or N-C₁₋ ₄alkylpiperazine ring;
R¹⁷ represents hydrogen or C₁₋₆alkyl;
R¹⁸ represents hydrogen, C₁₋₆alkyl, aryl, arylC₁₋₄alkyl, COR¹² or R¹⁷ and R¹⁸ together with the nitrogen atom to which they are attached form a morpholine, piperazine or N-C₁₋₄alkylpiperazine ring;
A represents CH₂ or C=O;
m represents zero or 1;
n represents 1,2 or 3;
X represents S or NH, or when R⁷ represents amino then X may also represent O;
Y represents O or S; with the proviso that when R¹ represents C₁₋₆alkyl and R⁵ represents hydrogen then R⁴ cannot represent nitro or NH₂.

3. A compound according to Claim 1 or Claim 2 in which R¹ represents a C₁₋₃alkyl group.

4. A compound according to any preceding claim in which R³ represents a C₂₋₃alkyl group.

5. A compound according to any preceding claim in which R⁴ is a group linked to the benzene ring of the rest of the molecule via a nitrogen atom.

6. A compound according to Claim 5 in which R⁴ represents NHCOR¹¹ (where R¹¹ is methyl, p-fluorophenyl, 2-thienyl or trifluoromethyl), NH₂, NHSO₂CH₃, NHCO₂CH₃, NHCO₂Et, NHCONHEt or NHCSNHEt.

7. A compound according to any one of Claims 1 to 4 in which R⁴ is a thienyl, thiazolyl or 1,2,4-triazolyl ring each substituted by methyl or aryl.

8. Compounds of formula (Ia) 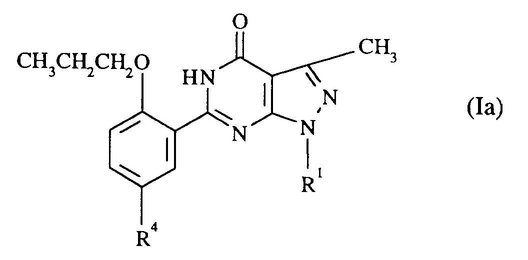 and physiologically acceptable salts and solvates (e.g. hydrates) thereof, in which R¹ represents C₁₋₃alkyl and R⁴ represents NHCOR¹¹ (where R¹¹ is methyl, p-fluorophenyl, 2-thienyl or trifluoromethyl, NHSO₂CH₃, NHCO₂CH₃, NHCO₂Et, NHCONHEt, NHCSNHEt or a thienyl, thiazolyl or 1,2,4-triazolyl ring each substituted by methyl or aryl.

9. 1,3-Dimethyl-6-(2-propoxy-5-acetamidophenyl)-1,5-dihydropyrazolo[3,4-d]pyrimidin-4-one;
1-ethyl-3-methyl-6-[2-propoxy-5-(4-methyl-2-thiazolyl)phenyl]-1,5-dihydropyrazolo[3,4-d]pyrimidin-4-one;
1-ethyl-3-methyl-6-[2-propoxy-5-(2-methyl-4-thiazolyl)phenyl]-1,5-dihydropyrazolo[3,4-d]pyrimidin-4-one;
1-ethyl-3-methyl-6-[2-propoxy-5-(2-(3-pyridyl)-4-thiazolyl)phenyl]-1,5-dihydropyrazolo[3,4-d]pyrimidin-4-one;
1,3-dimethyl-6-[2-propoxy-5-(2-methyl-4-thiazolyl)phenyl]-1,5-dihydropyrazolo[3,4-d]pyrimidin-4-one;
1,3-dimethyl-6-[2-propoxy-5-(3-phenyl-1,2,4-triazol-5-yl)phenyl]-1,5-dihydropyrazolo[3,4-d]pyrimidin-4-one;
1,3-dimethyl-6-(2-propoxy-5-methanesulfonamidophenyl)-1,5-dihydro-pyrazolo[3,4-d]pyrimidin-4-one; and physiologically acceptable salts and solvates (e.g. hydrates) thereof.

10. Compounds of formula (I) and salts and solvates (e.g. hydrates) thereof in which:
R¹ represents C₁₋₆alkyl, 2,2,2-trifluoroethyl or benzyl;
R² represents methyl;
R³ represents C₂₋₄alkyl;
R⁴ represents nitro, cyano, C₁₋₆alkoxy, C(=X)NR⁶R⁷, NR⁸R⁹, (CH₂)mNR¹⁰C(=Y)R¹¹ or thiazolyl or 1,2,4-triazolyl each ring optionally substituted by a C₁₋₄alkyl or aryl group;
R⁵ represents hydrogen or C₁₋₆alkyl;
R⁶ represents hydrogen or C₁₋₆alkyl;
R⁷ represents, hydrogen, amino, hydroxyl, C₁₋₆alkyl, aryl or arylC₁₋ ₄alkyl;
R⁸ represents hydrogen or C₁₋₆alkyl;
R⁹ represents hydrogen, C₁₋₆alkyl, SO₂R¹², CO₂R¹², C(=NCN)SR¹² or C(=NCN)NR¹³R¹⁴;
R¹⁰ represents hydrogen or C₁₋₆alkyl;
R¹¹ represents C₁₋₆alkyl optionally substituted by one or more halogen atoms, or R¹¹ represents aryl, arylC₁₋₄alkyl, thienyl, NR¹⁵R¹⁶, CH₂NR¹⁷R¹⁸ or R¹⁰ and R¹¹ together represent -A(CH₂)ₙ-;
R¹² represents C₁₋₆alkyl, aryl or arylC₁₋₄alkyl;
R¹³ represents hydrogen or C₁₋₆alkyl;
R¹⁴ represents hydrogen, C₁₋₆alkyl, aryl, arylC₁₋₄alkyl or R¹³ and R¹⁴ together with the nitrogen atom to which they are attached form a morpholine, piperazine or N-C₁₋₄alkylpiperazine ring;
R¹⁵ represents hydrogen or C₁₋₆alkyl or R¹⁰ and R¹⁵ together represent -A(CH₂)ₙ-;
R¹⁶ represents hydrogen, C₁₋₆alkyl, aryl, arylC₁₋₄alkyl, CO₂R¹², CH₂CO₂R¹² or R¹⁵ and R¹⁶ together with the nitrogen atom to which they are attached form a morpholine, piperazine or N-C₁₋ ₄alkylpiperazine ring;
R¹⁷ represents hydrogen or C₁₋₆alkyl;
R¹⁸ represents hydrogen, C₁₋₆alkyl, aryl, arylC₁₋₄alkyl, COR¹² or R¹⁷ and R¹⁸ together with the nitrogen atom to which they are attached form a morpholine, piperazine or N-C₁₋₄alkylpiperazine ring;
A represents CH₂ or C=O;
m represents zero or 1;
n represents 1,2 or 3;
X represents S or NH, or when R⁷ represents amino then X may also represent O;
Y represents O or S, with the proviso that when R¹ represents C₁₋₆alkyl and R⁵ represents hydrogen then R⁴ cannot represent nitro or NH₂.

11. A pharmaceutical composition comprising a compound of formula (I) or a physiologically acceptable salt or solvate thereof (as defined in Claim 1) together with a pharmaceutically acceptable diluent or carrier.

12. A process for the preparation of a compound of formula (I) or a salt or solvate thereof (as defined in Claim 2) which comprises:
(A) for preparing a compound in which R⁴ represents nitro or C₁₋₆alkoxy, cyclising a compound of formula (II) 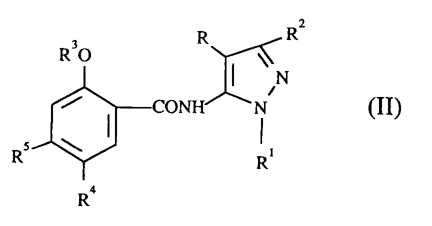 in which R¹, R², R³ and R⁵ are as defined in Claim 1, R⁴ is nitro or C₁₋₆alkoxy and R is CN or CONH₂;
(B) converting a compound of formula (I) to a different compound of formula (I); or
(C) reacting a compound of formula (I) in which R⁴ is a reactive atom or group to introduce the desired R⁴ group; together, if desired or appropriate, with salt formation or conversion of one salt to another salt as an optional step subsequent to process (A), (B) or (C).

13. Use of a compound of formula (I) or a physiologically acceptable salt or solvate thereof (as defined in Claim 1) for the manufacture of a medicament for the treatment of stable, unstable and variant (Prinzmetal) angina, hypertension, pulmonary hypertension, congestive heart failure, renal failure, atherosclerosis, conditions of reduced blood vessel patency, peripheral vascular disease, stroke, bronchitis, chronic asthma, allergic asthma, allergic rhinitis, glaucoma or diseases characterised by disorders of gut motility.
